# EUROPEAN PATENT SPECIFICATION

(11) **EP 3 539 525 B1**
(45) Date of publication and mention of the grant of the patent: **13.11.2024**
(21) Application number: 18792082.2
(22) Date of filing: 25.04.2018
(51) Int. Cl.: A61H 1/02, G06F 3/0481, A61B 5/00, G06F 3/16, G06F 3/01, A61B 5/11, G16H 20/30, G09B 5/06, G09B 19/00, G06F 3/04842, G10L 15/00

(54) **REHABILITATION ASSISTANCE SYSTEM, REHABILITATION ASSISTANCE METHOD, AND REHABILITATION ASSISTANCE PROGRAM**
REHABILITATIONSUNTERSTÜTZUNGSSYSTEM, REHABILITATIONSUNTERSTÜTZUNGSVERFAHREN UND REHABILITATIONSUNTERSTÜTZUNGSPROGRAMM
SYSTÈME, PROCÉDÉ ET PROGRAMME D'AIDE À LA RÉÉDUCATION

(30) Priority: 25.04.2017 JP 2017086674; 23.10.2017 JP 2017204243
(43) Date of publication of application: 18.09.2019
(73) Proprietor: Medivr, Inc., Toyonaka City, Osaka 561-0872 (JP)
(72) Inventor: HARA Masahiko, Toyonaka City, Osaka 561-0872 (JP)
(74) Representative: Betten & Resch
(86) International application number: PCT/JP2018/016886
(87) International publication number: WO 2018/199196

(56) References cited:
- EP-A1- 3 153 146
- EP-A1- 3 165 208
- WO-A1-2016/002885
- JP-A- 2008 000 230
- JP-A- 2010 131 321
- JP-A- 2011 110 215
- JP-A- 2015 041 097
- JP-A- H0 353 119
- US-A1- 2011 112 441

## Description

### TECHNICAL FIELD

The present invention relates to a rehabilitation assistance system, a rehabilitation assistance method, and a rehabilitation assistance program.

### BACKGROUND ART

In the above technical field, patent literature 1 discloses a system configured to assist rehabilitation performed for a hemiplegia patient suffering apoplexy or the like.

### CITATION LIST

### PATENT LITERATURE

Patent literature 1: Japanese Patent Laid-Open No. 2015-228957
Patent literature 2: EP 3 153 146 A1
Patent literature 3: WO 2016/002885 A1
Patent literature 4: EP 3 165 208 A1
Patent literature 5: US 2011/112441 A1
EP 3 153 146 A1 discloses first display apparatus, which is to be attached to the head of a patient, and enables a body portion of the patient to be seen. A display controller causes an index image that functions as an index for a rehabilitation motion, to be displayed on the first display apparatus.
WO 2016/002885 A1 and EP 3 165 208 A1 disclose a rehabilitation assistance device provided with: an arm part having a holding part for holding a part of the upper limb or the lower limb of a user, the arm part movably supporting the holding part; a memory unit for storing training information, the memory unit storing first training information defined in advance; a motion information acquisition unit for acquiring motion information associated with the movement of the holding part, the motion information acquisition unit acquiring first motion information for the holding part moved on the basis of the first training information; a motion evaluation unit for generating evaluation information in which the motion information is evaluated, the motion evaluation unit evaluating the first motion information and generating first evaluation information; and a display for displaying at least one of the training information, the motion information, and the evaluation information.
US 2011/112441 A1 discloses a method and apparatus to perform combined cognitive and motor rehabilitation on a computerized non-portable system or on single portable device. A patient can play a variety of games that require the patient to perform a variety of memory exercises which involve physical exertion. The activities of the patient are monitored with pattern analysis software which provides feedback to the patient. The feedback can include voice synthesis, video guidance, progression messages etc. Patient data obtained while the patient is performing each of the memory exercises is stored locally on a database module and then uploaded to a cloud server. A remote psychologist/psychiatrist monitors the patient by logging into the same cloud, and updating cognition exercises. The same therapist can have live chats with the patient for further interaction and coaching.

### TECHNICAL PROBLEM

In the technique described in the above patent literature 1, however, it is impossible to perform active target updating according to an action of a user, and the same load needs to be repeated for any user.

The present invention enables to provide a technique of solving the above-described problem.

### SUMMARY OF THE INVENTION

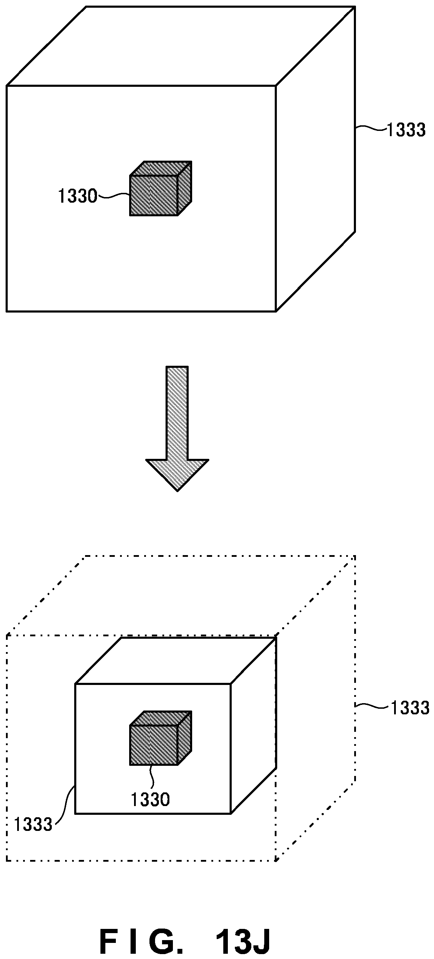

The present invention is defined in claim 1. The dependent claims define embodiments of the invention. The invention as claimed is best understood in light of the embodiment described in the context of Figure 13J. Other embodiments described herein do not necessarily describe the complete combination of features as claimed, but are useful for understanding the invention as claimed.

### ADVANTAGEOUS EFFECTS OF INVENTION

According to the present invention, it is possible to perform active target updating according to the rehabilitation action of a user.

### BRIEF DESCRIPTION OF DRAWINGS

Fig. 1 is a block diagram showing the arrangement of a rehabilitation assistance system according to the first example embodiment;
Fig. 2 is a block diagram showing the arrangement of a rehabilitation assistance system according to the second example embodiment;
Fig. 3 is a view showing a display screen example of the rehabilitation assistance system according to the second example embodiment;
Fig. 4 is a view showing a display screen example of the rehabilitation assistance system according to the second example embodiment;
Fig. 5 is a flowchart showing the procedure of processing of the rehabilitation assistance system according to the second example embodiment;
Fig. 6 is a view showing a display screen example of the rehabilitation assistance system according to the second example embodiment;
Fig. 7 is a view showing a display screen example of the rehabilitation assistance system according to the second example embodiment;
Fig. 8 is a view showing a display screen example of the rehabilitation assistance system according to the second example embodiment;
Fig. 9 is a view showing the other example of the rehabilitation assistance system according to the second example embodiment;
Fig. 10 is a view showing the arrangement of a database of the rehabilitation assistance system according to the second example embodiment;
Fig. 11 is a view showing a display screen example of the rehabilitation assistance system according to the second example embodiment;
Fig. 12 is a view showing a display screen example of the rehabilitation assistance system according to the second example embodiment;
Fig. 13A is a view for explaining the outline of the operation of the rehabilitation assistance system according to the second example;
Fig. 13B is a view for explaining the outline of the operation of the rehabilitation assistance system according to the second example embodiment;
Fig. 13C is a view for explaining the outline of the operation of a rehabilitation assistance system according to the third example embodiment;
Fig. 13D is a view for explaining the arrangement position of a visual recognition support image in the rehabilitation assistance system according to the third example embodiment;
Fig. 13E is a view for explaining another example of the visual recognition support image in the rehabilitation assistance system according to the third example embodiment;
Fig. 13F is a view for explaining still another example of the visual recognition support image in the rehabilitation assistance system according to the third example embodiment;
Fig. 13G is a view for explaining still another example of the visual recognition support image in the rehabilitation assistance system according to the third example embodiment;
Fig. 13H is a view for explaining still another example of the visual recognition support image in the rehabilitation assistance system according to the third example embodiment;
Fig. 13I is a view for explaining still another example of the visual recognition support image in the rehabilitation assistance system according to the third example embodiment;
Fig. 13J is a view for explaining still another example of the visual recognition support image in the rehabilitation assistance system according to the claimed invention;
Fig. 14 is a block diagram for explaining the arrangement of the rehabilitation assistance system according to the third example embodiment of the present invention;
Fig. 15A is a view for explaining an example of a patient table provided in a rehabilitation assistance server included in the rehabilitation assistance system according to the third example embodiment of the present invention;
Fig. 15B is a view for explaining an example of a display parameter table provided in the rehabilitation assistance server included in the rehabilitation assistance system according to the third example embodiment of the present invention;
Fig. 15C is a view for explaining an example of an image table provided in the rehabilitation assistance server included in the rehabilitation assistance system according to the third example embodiment of the present invention;
Fig. 16 is a block diagram for explaining the hardware arrangement of the rehabilitation assistance server included in the rehabilitation assistance system according to the third example embodiment;
Fig. 17A is a flowchart for explaining the processing procedure of the rehabilitation assistance server included in the rehabilitation assistance system according to the third example embodiment;
Fig. 17B is a flowchart for explaining the processing procedure of visual recognition support image display of the rehabilitation assistance server included in the rehabilitation assistance system according to the third example embodiment;
Fig. 18 is a block diagram for explaining the arrangement of a rehabilitation assistance system according to the fourth example embodiment;
Fig. 19 is a view for explaining an example of a sound table provided in a rehabilitation assistance server included in the rehabilitation assistance system according to the fourth example embodiment;
Fig. 20 is a view for explaining the hardware arrangement of the rehabilitation assistance server included in the rehabilitation assistance system according to the fourth example embodiment;
Fig. 21A is a flowchart for explaining the processing procedure of the rehabilitation assistance server included in the rehabilitation assistance system according to the fourth example embodiment;
Fig. 21B is a flowchart for explaining the processing procedure of sound output control of the rehabilitation assistance server included in the rehabilitation assistance system according to the fourth example embodiment;
Fig. 22 is a view for explaining the control method of a rehabilitation assistance system according to the fifth example embodiment;
Fig. 23 is a view for explaining the control method of the rehabilitation assistance system according to the fifth example embodiment;
Fig. 24 is a view showing a display screen example of the rehabilitation assistance system according to the fifth example embodiment;
Fig. 25 is a view showing a display screen example of the rehabilitation assistance system according to the fifth example embodiment; and
Fig. 26 is a view showing a display screen example of the rehabilitation assistance system according to the fifth example embodiment.

### DESCRIPTION OF EXAMPLE EMBODIMENTS

Example embodiments will now be described in detail with reference to the drawings.

### [First Example Embodiment]

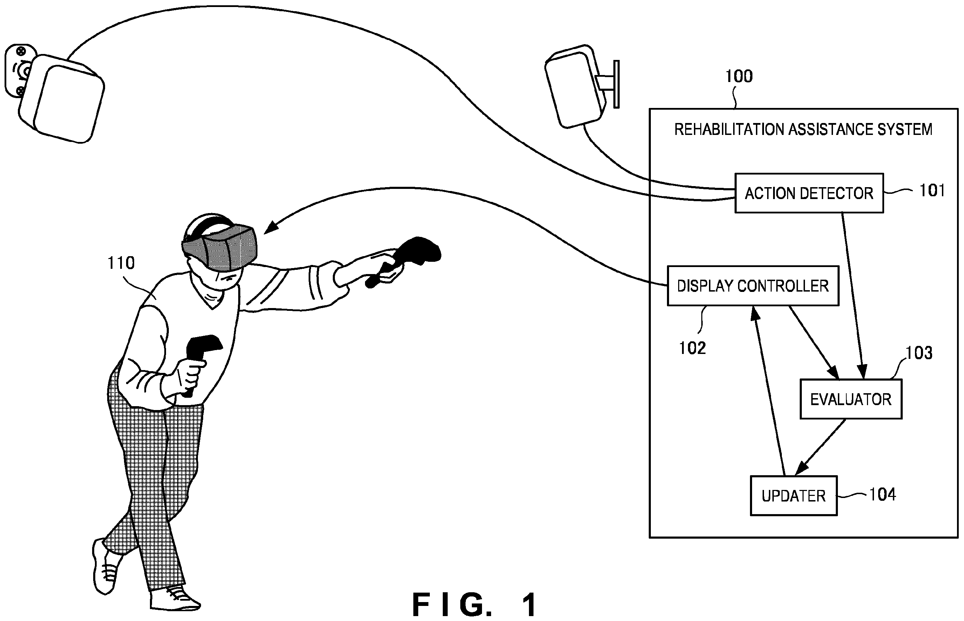

A rehabilitation assistance system 100 according to the first example embodiment of the present invention will be described with reference to Fig. 1.

As shown in Fig. 1, the rehabilitation assistance system 100 includes an action detector 101, a display controller 102, an evaluator 103, and an updater 104.

The action detector 101 detects a rehabilitation action of a user 110. The display controller 102 displays an avatar image that moves in accordance with the detected rehabilitation action and a target image representing the target of the rehabilitation action.

The evaluator 103 evaluates the rehabilitation ability of the user in accordance with the difference between the rehabilitation action and a target position represented by the target image. The updater 104 updates the target position in accordance with the evaluation result by the evaluator 103.

The action detector 101 further detects a second rehabilitation action of the user during a first rehabilitation action. When a predetermined or more evaluation is made only for the first rehabilitation action, the evaluator 103 evaluates the rehabilitation ability based on both the first rehabilitation action and the second rehabilitation action. This makes it possible to perform active and proper target updating according to the rehabilitation action of the user.

### [Second Example Embodiment]

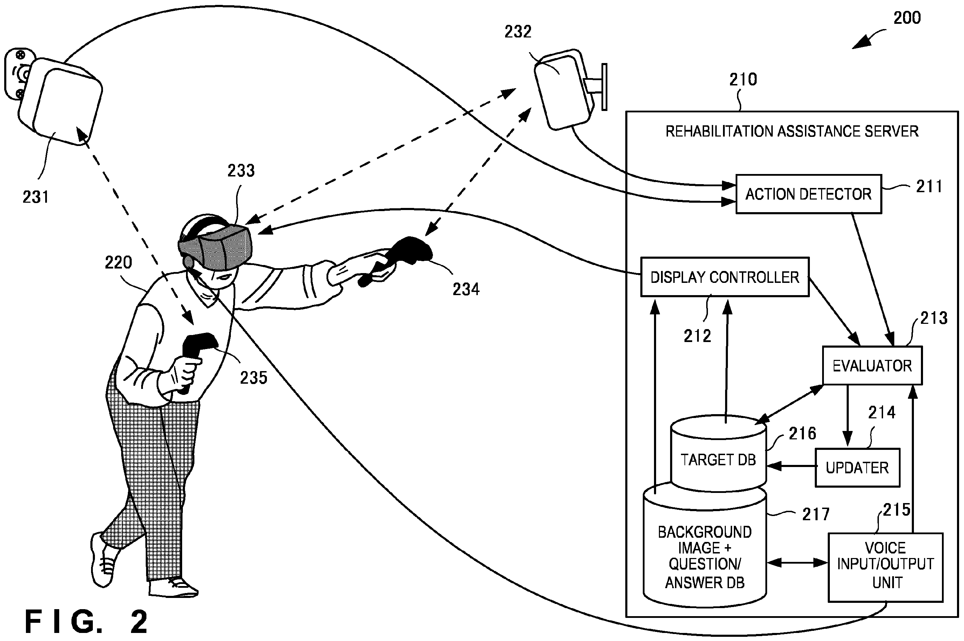

A rehabilitation assistance system 200 according to the second example embodiment of the present invention will be described next with reference to Fig. 2. Fig. 2 is a view for explaining the arrangement of the rehabilitation assistance system according to this example embodiment.

As shown in Fig. 2, the rehabilitation assistance system 200 includes a rehabilitation assistance server 210, two base stations 231 and 232, a head mounted display 233, and two controllers 234 and 235. Note that the head mounted display 233 can be any one of a nontransparent type, a video see-through type, and an optical see-through type.

In addition, the rehabilitation assistance server 210 includes an action detector 211, a display controller 212, an evaluator 213, an updater 214, a voice input/output unit 215, a target database 216, and a background image + question/answer database 217.

The action detector 211 acquires the positions of the controllers 234 and 235 in the hands of a user 220 and the position of the head mounted display 233 via the base stations 231 and 232, and detects the rehabilitation action of the user 220 based on changes in the positions.

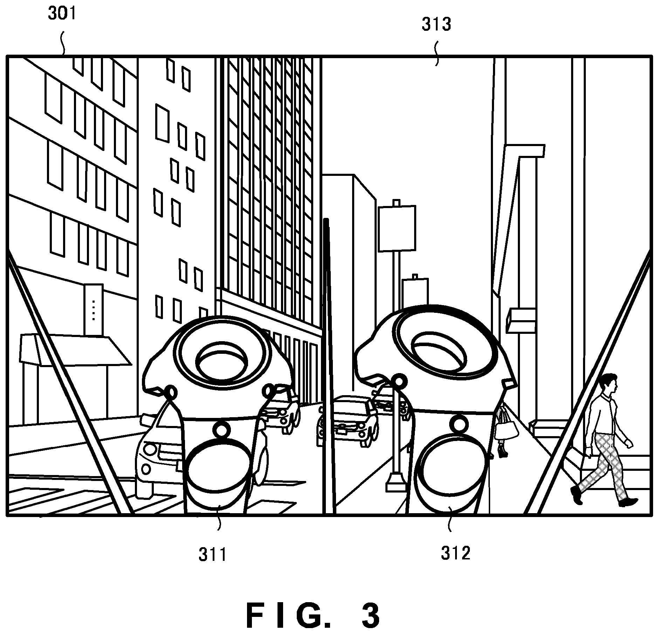

The display controller 212 causes the head mounted display 233 to display an avatar image that moves in accordance with the detected rehabilitation action and a target image representing the target of the rehabilitation action. Fig. 3 is a view showing an example of avatar images 311 and 312 in a screen 301 displayed on the head mounted display 233. The avatar images 311 and 312 are displayed on a background image 313 in a superimposed manner. In this example, the avatar images 311 and 312 have the same shapes as the controllers 234 and 235 and move in the screen 301 in accordance with the motions of the controllers 234 and 235. Additionally, a background image 313 changes depending on the position and orientation of the head mounted display 233. As shown on the avatar images 311 and 312, buttons are prepared on the controllers 234 and 235, and the controllers 234 and 235 are configured to be able to do various kinds of setting operations and the like. Here, a landscape video (for example, a movie obtained by capturing a street in New York) obtained by capturing an actual landscape is displayed as the background image 313. As the landscape video, a video of a road around the rehabilitation facility may be used. This makes the user feel to take a walk in a foreign country or feel to stroll in a familiar place. When the landscape video is superimposed, training in an enormous information amount can be implemented while entertaining the patient.

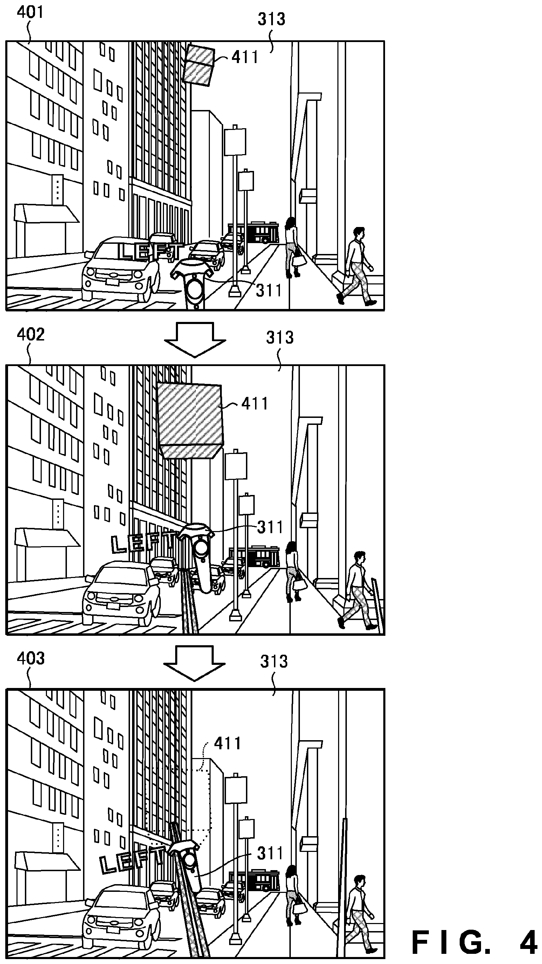

In addition, for example, as shown in Fig. 4, the display controller 212 displays an object 411 superimposed on the background image 313 in screens 401 to 403 of the head mounted display 233. The object 411 is displayed while gradually changing its display position and size such that it appears to be falling downward from overhead of the user 220. The user 220 moves the controllers 234 and 235 to bring the avatar image 311 in the screen close to the object 411. When the avatar image 311 hits the object 411, the object 411 disappears. In the screens 401 to 403, characters "left" near the avatar image 311 of the sensor means touching the object 411 with the left hand.

The evaluator 213 compares the rehabilitation action detected by the action detector 211 and the target position represented by the target image displayed by the display controller 212, and evaluates the rehabilitation ability of the user. More specifically, the evaluator 213 decides, by comparing the positions in a three-dimensional virtual space, whether the avatar image 311 that moves in correspondence with the rehabilitation action detected by the action detector 211 overlaps the object 411 serving as the target image. As a result, if these overlap, the evaluator 213 evaluates that one rehabilitation action is completed, and adds a point. As for the position of the object 411 in the depth direction, various steps (for example, three steps) are prepared and set to different points (a high point for a far object, and a low point for a close object), respectively.

The updater 214 updates the target task in accordance with the integrated point. For example, the target task may be updated using a task achievement ratio (number of achieved targets/number of tasks) or the like.

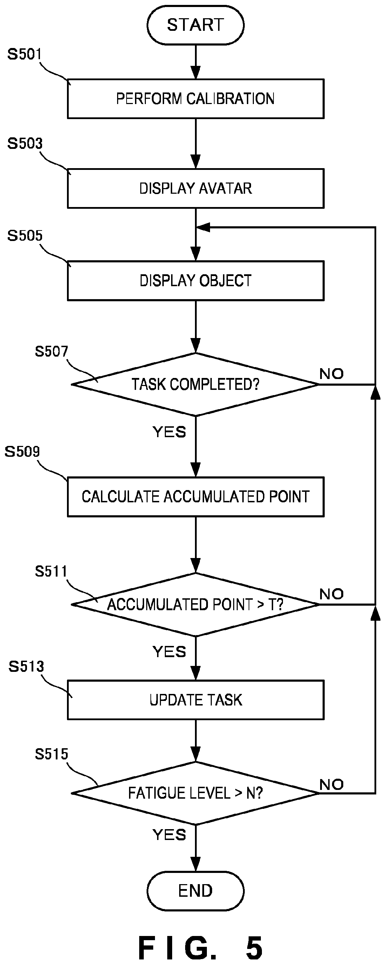

Fig. 5 is a flowchart showing the procedure of processing in the rehabilitation assistance server 210. In step S501, as calibration processing, the target of the rehabilitation action is initialized in accordance with the user. More specifically, each patient is first caused to do a work in an action range as calibration, it is set to the initial value, and the target is initialized in accordance with the user.

In addition, a target according to the attribute information (for example, whether the user is an athlete or suffers from the Parkinson disease) of the user is set by referring to the target database 216. For example, in a case of an injured athlete, an initial value not to make the injury worse is set. In a case of a user suffering from the Parkinson disease, an exercise to make the disease progress slow is set to the initial value. Furthermore, each patient is first caused to do a work in an action range, it is set to the initial value, and the target is initialized in accordance with the user.

Next, in step S503, the avatar images 311 and 312 are displayed in accordance with the positions of the controllers 234 and 235 detected by the action detector 211. Furthermore, in step S505, the object 411 is displayed at a position and speed according to the set task.

In step S507, the motions of the avatar images 311 and 312 and the motion of the object 411 are compared, and it is determined whether the task is completed. If the task is not completed, the process directly returns to step S505, and the next object is displayed without changing the difficulty of the task.

If the task is completed, the process advances to step S509 to calculate an accumulated point, a task achievement probability, and the like. The process further advances to step S511 to compare the accumulated point, the task achievement probability, or the like with a threshold T. If the accumulated point, the task achievement probability, or the like exceeds the predetermined threshold T, the process advances to step S513 to update the exercise intensity of the task. If the accumulated point, the task achievement probability, or the like does not reach the threshold T, the process returns to step S505, and the next object is displayed without changing the difficulty of the task.

For example, when the achievement level in a short range exceeds 80% (or a count such as 10 times may be used), the display frequency of an object in a middle range is raised. When the achievement level of the object in the middle range exceeds 80% (or a count such as 10 times may be used), the display frequency of an object in a long range is raised. Conversely, if the achievement level is low, the target value may be set to the short range.

As for the task updating here as well, the task is changed in accordance with the attribute of the user (for example, whether the user is an injured athlete or a patient suffering from the Parkinson disease). As the task updating method, a method of switching the background image is also conceivable.

After the task is updated, the process advances to step S515, and the fatigue level of the user is calculated and compared with a threshold N. If the fatigue level exceeds the predetermined threshold, the "stop condition" is satisfied, and the processing is ended. For example, (fatigue level = 1 - collection ratio of closest objects) can be calculated. Alternatively, (fatigue level = 1/eye motions) may be calculated. If it is obvious that the user is not concentrating (for example, the user is not searching for an object at all or does not move the head), it would be meaningless to continue the rehabilitation any more, and the user takes a break. In addition, the fatigue level may be calculated by detecting a decrease in the speed (acceleration) of stretching out the hand.

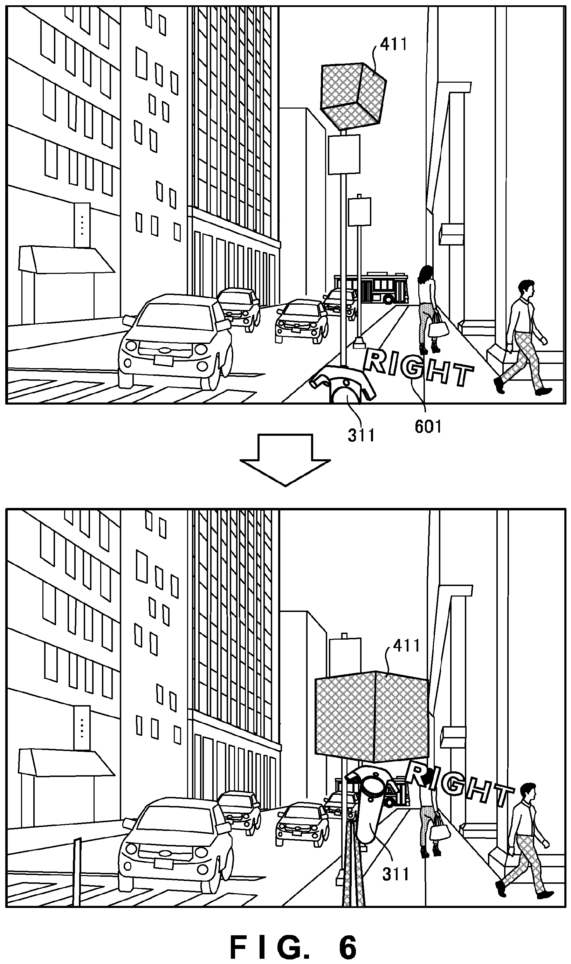

Additionally, for example, when the accumulated point exceeds a predetermined threshold, which one of the two, left and right controllers 234 and 235 should be used to touch the object 411 (right here) is instructed, as indicated by a character image 601 shown in Fig. 6. This requires a cognitive function of recognizing a character, and also, the difficulty of the action rises, and an advanced motor function is necessary. That is, a dual task for the cognitive function and the motor function is required.

Note that in Fig. 6, the instruction is made using a character. However, the present invention is not limited to this, and the instruction may be made by an arrow, a color, or a voice. As described above, in this example embodiment, the load is updated in accordance with the evaluation of the rehabilitation action.

### (Dual Task)

An able-bodied person makes two or more actions simultaneously, for example, "walks while talking" in a daily life. Such "an ability to make two actions simultaneously" declines with age. For example, "stop when talked to during walking" occurs. It is considered that an elderly person falls not only because of "the deterioration of the motor function" but also because of involvement of such "decline in the ability to make two actions simultaneously". In fact, there are many elderly persons who are judged to have sufficiently recovered the motor function by rehabilitation but fall after returning to the home. One factor responsible to this is that the rehabilitation is performed in a state in which the environment and conditions to allow a person to concentrate on the rehabilitation action are organized. That is, a living environment includes factors that impede concentration on an action, and an action is often made under a condition that, for example, the view is poor, an obstacle exists, or consciousness is turned to a conversation.

Hence, it is considered that it is important to perform such rehabilitation that makes the user distract attention. It is preferable to give a specific dual task and perform training. Such a dual task training is an effective program not only to prevent a fall of an elderly person but also to prevent dementia.

The dual task training includes not only a training that combines a cognitive task and an exercise task but also a training that combines two types of exercise tasks.

As cognitive task + exercise task, a training such as walking while subtracting one by one from 100 can be performed. As exercise task + exercise task, a training such as walking without spilling water from a glass can be performed.

In a case in which the walking speed is lower about 20% in a dual task walking test than in simple walking, the evaluator 213 evaluates that the risk of fall is high, and notifies the display controller 212 to repeat the dual task.

Note that the dual task is readily more effective to "a person having a relatively high moving ability". For example, for an elderly person who cannot move without a stick even indoors, strengthening the balance ability (muscle power, sense of equilibrium, and the like) is given higher priority than the dual task ability. Roughly judging, it can be expressed that the dual task ability is important for a person requiring support, and the balance ability other than the dual task ability is important for a person requiring care. A time-series change in calibration is displayed, and the improvement of the exercise range of the user is visually displayed.

### (Setting Change by User Attribute)

For a patient expected to normally improve (a patient suffering from an orthopedic disease such as a bone fracture and assumed to completely improve), hardest rehabilitation actions are set to speed up the improvement.

For a patient whose degree of improvement changes individually (in a case of brain infarction or the like, a paralysis of a different form occurs depending on the morbid portion), the load of a task is improved to some extent, and the improvement of the load is stopped at a certain level.

In a case of a patient suffering from hypofunction in principle due to the Parkinson disease or the like, periodically evaluating the current exercise enable state is useful.

### (Other Examples of Dual Task Training)

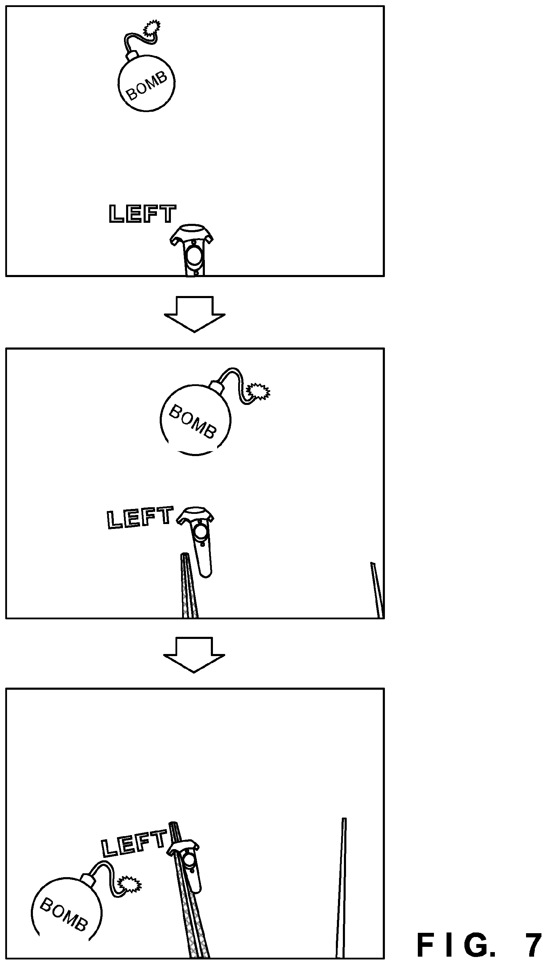

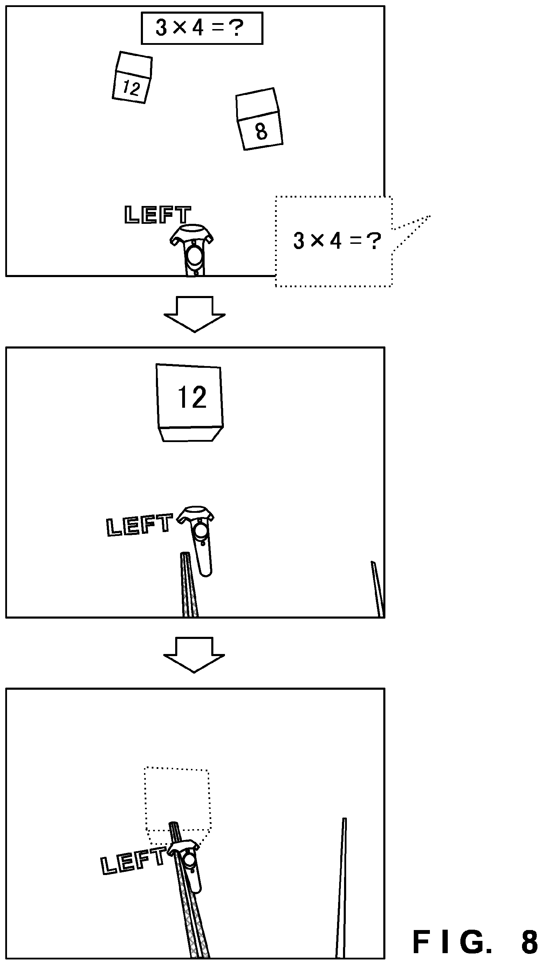

Fig. 7 is a view showing another example of an image for dual task training. A loser (bomb) is mixed among objects, thereby requiring the cognitive function. Alternatively, as shown in fig. 8, a question image (for example, multiplication, here) may be displayed on the background screen in a superimposed manner, and only acquisition of an object on which a correct answer is displayed may be evaluated. One of rock, scissors, and paper may be displayed on the background screen, and the user may be requested to collect an object on which a mark to win is displayed.

In addition, a number may be simply displayed on each object, and only acquisition of an object of a large number may be evaluated. Alternatively, a traffic signal may be displayed in the background image 313, and when the user acquires an object at red light, the evaluator 213 may decrement the point.

According to this example embodiment, since the task is updated in accordance with the achievement level (for example, achievement probability) of the rehabilitation action, a load according to the degree of progress of rehabilitation can be given to the user. In addition, when the background image 313 is displayed, the patient can enjoy and also perform rehabilitation in a situation in which he/she turns consciousness to the periphery, and can implement a safer life when returning to the physical world.

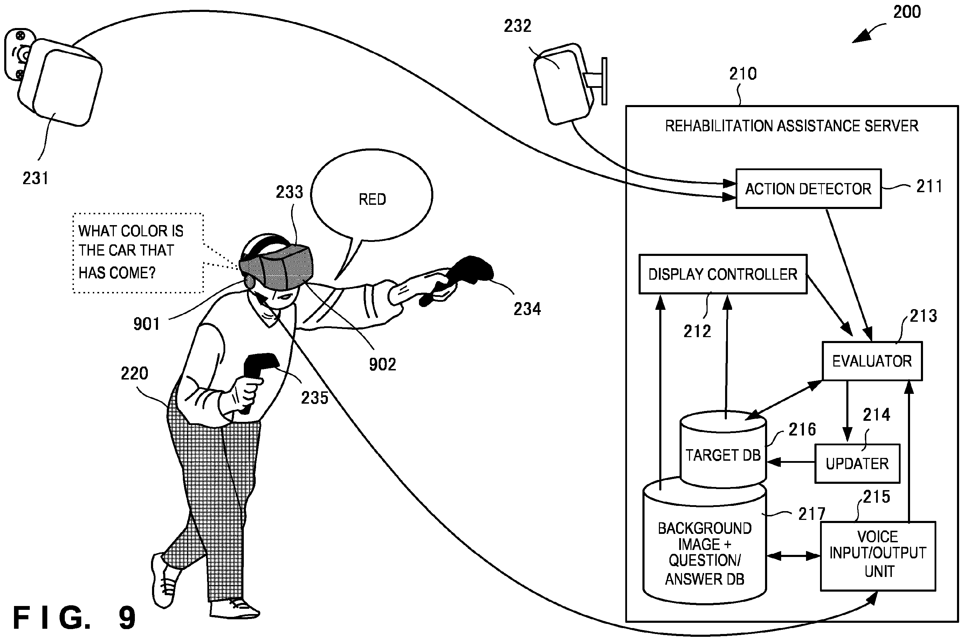

Fig. 9 is a view showing still another example of dual task training. As shown in Fig. 9, the voice input/output unit 215 outputs a question voice concerning the background image to a headphone 901 and acquires an answer to the question via a microphone 902 provided on the head mounted display 233. The evaluator 213 performs voice recognition processing for the answer acquired as voice information, compares the answer with an answer prepared in advance, and evaluates the rehabilitation ability of the user in accordance with the comparison result.

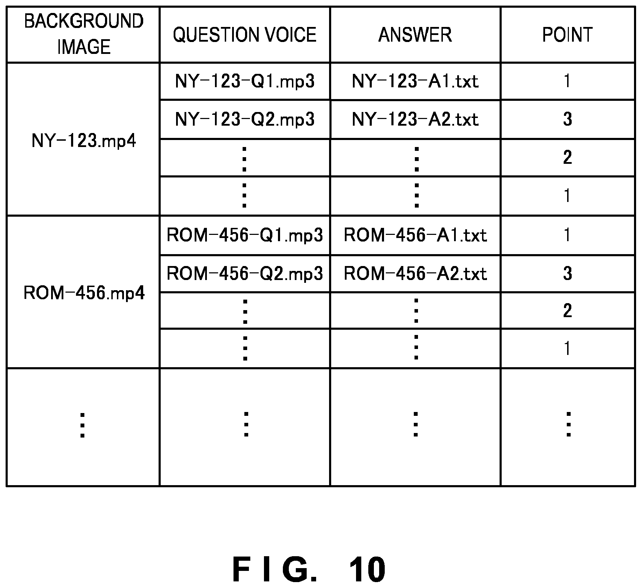

Fig. 10 is a view showing an example of the contents of the background image + question/answer database 217. A question voice, an answer, and a point are stored in association with a background movie.

As a reaction of the user, a result that the object collection ratio lowers is expected in a dual task. A result that the object collection achievement ratio does not change even when the dual task is displayed is expected as a goal. The object collection ratio or object reach ratio in a single task is compared with that in a dual task, and training is repetitively performed until the difference falls within a predetermined range.

Dual task training that simultaneously requires a motor function and a cognitive function has been described above. However, the present invention is not limited to this, and dual task training that simultaneously requires two motor functions may be performed.

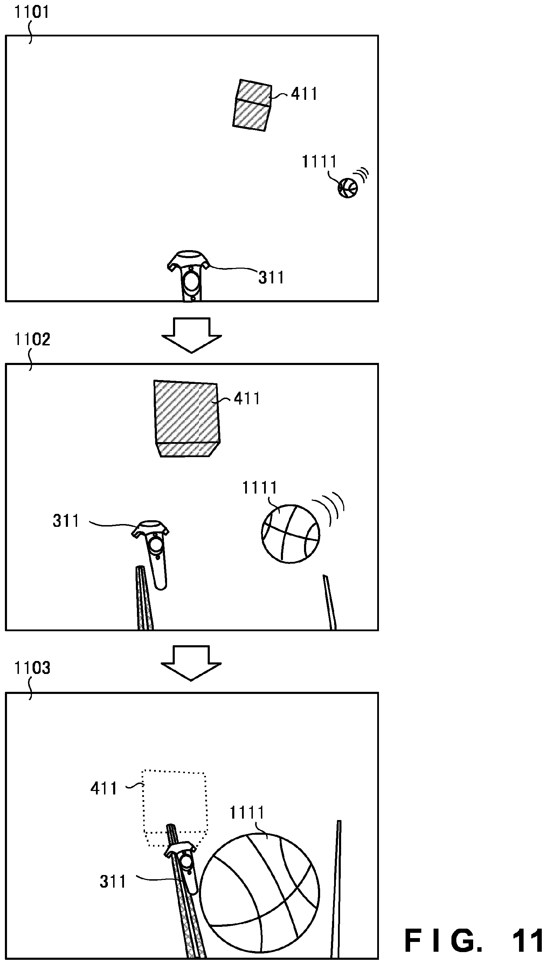

For example, as shown in Fig. 11, the user may be required to pick up the object 411 while getting out of the way of a flying object 1111. Whether the user has dodged the object 1111 well can be determined by detecting the position of a sensor provided on the head mounted display 233. Evaluation and task updating are performed based on the achievement points (for example, achievement ratios) of both of the two rehabilitation actions.

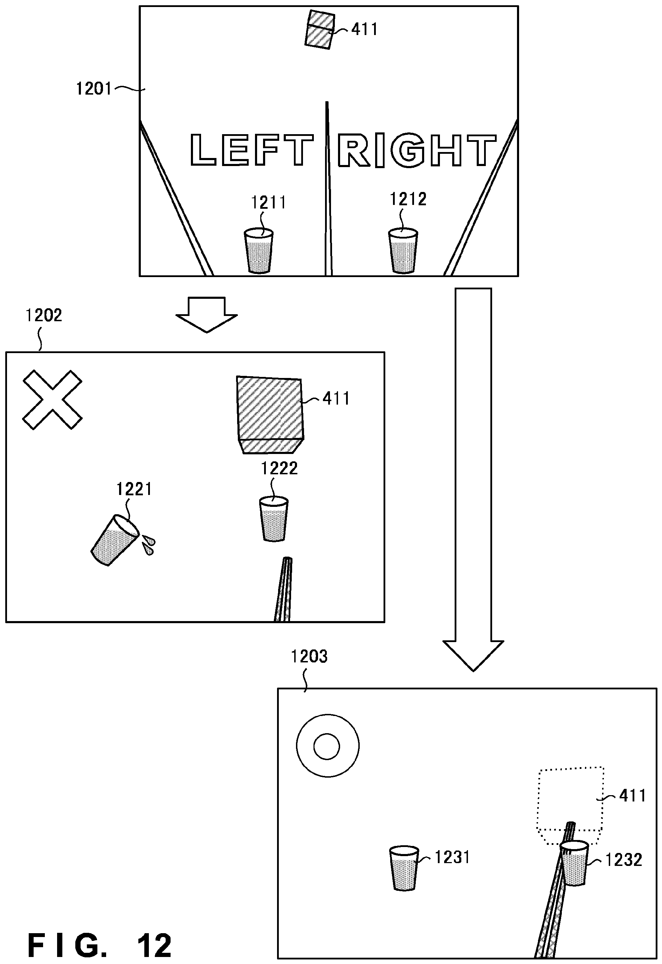

Additionally, for example, as indicated by an image 1201 shown in Fig. 12, glass images 1211 and 1212 with water may be displayed as avatar images that move in accordance with the actions of the controllers 234 and 235, and the object 411 may be collected by moving the glass images 1211 and 1212. However, as indicated by an image 1202, when a glass image 1221 is tilted, and water spills, a point cannot be obtained even when the object 411 is collected by a glass image 1222. A point is added only when the object 411 is collected without spilling water from the glass images 1231 and 1232, as indicated by an image 1203.

In addition, it can be considered that the user is required to cause the avatar image on the reverse side of the avatar image on the side of collecting the object to always touch a designated place. The user may be required to collect the object while pressing a designated one of the buttons provided on the controllers 234 and 235 a predetermined number of times. In addition, when a sensor configured to acquire the motion of a foot of the user is provided, the user may be required to move a designated foot.

### [Third Example Embodiment]

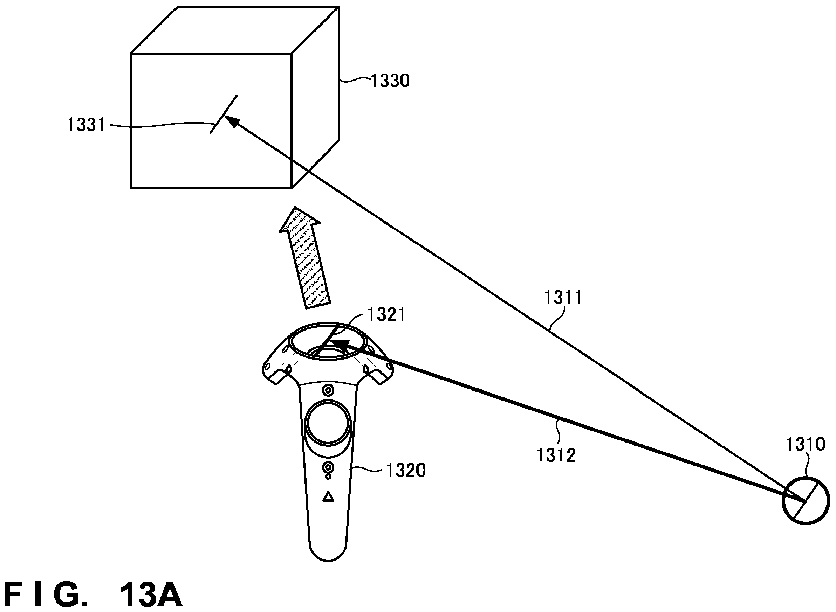

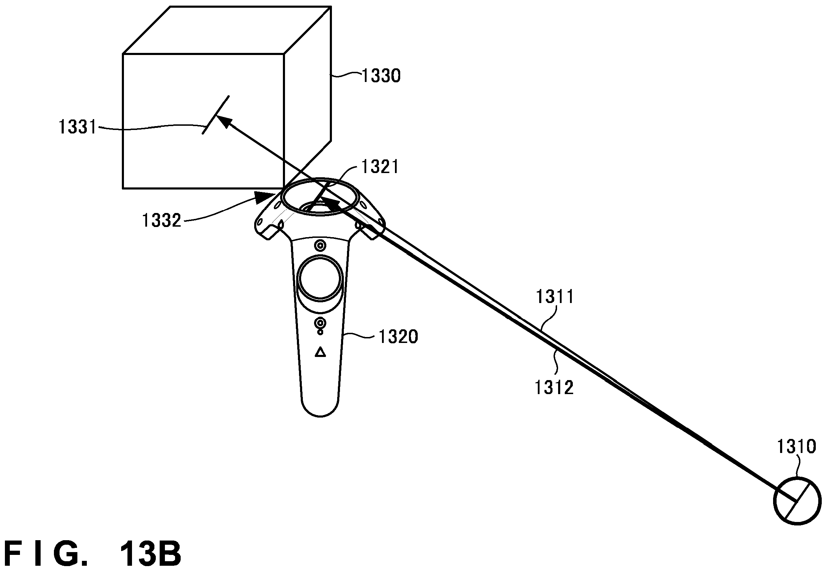

A rehabilitation assistance system according to the third example embodiment of the present invention will be described next with reference to Figs. 13A to 17B. Fig. 13A is a view for explaining the outline of the operation of the second example embodiment of the rehabilitation assistance system. Fig. 13B is a view for explaining the outline of the operation of the rehabilitation assistance system according to the second example embodiment. The rehabilitation assistance system according to this example embodiment is different from the above-described second example embodiment in that a visual recognition support image that improves the recognizability (for example, visibility) of a target image is displayed. The rest of the components and operations is the same as in the second example embodiment. Hence, the same reference numerals denote the same components and operations, and a detailed description thereof will be omitted.

In the second example embodiment, the moving distance of an avatar image 1320, that is, an exercise distance 1312 of a user 220 is measured based on the distance between a reference 1310 and the sensor of the avatar image 1320 (the head portion of the avatar image 1320). A target distance 1311 that is a distance required to move an arm or the like by the user 220 is decided based on the distance between the reference 1310 and a reference line 1331 of an object 1330 serving as a target image. As a rehabilitation exercise, the user 220 moves the avatar image 1320 and brings it close to the object 1330.

However, as shown in Fig. 13B, when the avatar image 1320 touches an apex 1332 of the object 1330, the system judges that one of rehabilitation actions of the user 220 has ended, and displays the new object 1330 as the next target.

The system provider side wants the avatar image 1320 to touch the object 1330 when the user 220 completely stretches out the arm as the rehabilitation exercise. However, if the size of the object 1330 is large (the distance between the apex and the reference line 1331 is long), it is determined that the avatar image 1320 touches the object 1330 when it just touches an edge of the object 1330. Hence, since the user 220 cannot move the arm by the initially assumed distance, the expected rehabilitation effect is difficult to obtain.

In addition, since the user 220 can touch the object 1330 before he/she completely stretches out the arm, the feeling of achievement or feeling of satisfaction cannot sufficiently be obtained, and the motivation to rehabilitation may lower.

In this case, the exercise distance 1312 that is the distance the avatar image 1320 has actually moved deviates from the target distance 1311 that is the distance the user 220 should move. For this reason, the user 220 cannot do the exercise through the exercise distance 1312 set before the start of the rehabilitation, and the effect obtained by the rehabilitation is less than the expected effect.

For example, the length of one side of the object 1330 is set to 20.0 cm, and a diameter 1321 of the sensor portion of the avatar image 1320 (the head portion of the avatar image 1320) is set to 5.0 cm. In this case, when the user 220 makes the avatar image 1320 touch not the reference line 1331 but the apex 1332 of the object 1330, an error of about 10.0 cm is generated between the target distance 1311 and the exercise distance 1312.

For this reason, since the user 220 does not move the avatar image 1320 by the exercise distance 1312 assumed before the start of the rehabilitation, the effect of the rehabilitation the user 220 should enjoy decreases.

On the other hand, if the object 1330 is made small such that the user 220 can touch the object 1330 by completely stretching out the arm, it becomes difficult for the user 220 to visually recognize the position of the object 1330 in the screen. If the object 1330 cannot be visually recognized, the rehabilitation cannot hold.

In this example embodiment, the sensor portion (reactive portion) of the avatar image 1320 is formed into a region smaller than the head portion of the avatar image 1320. This can decrease the deviation (error) between the target distance 1311 and the exercise distance.

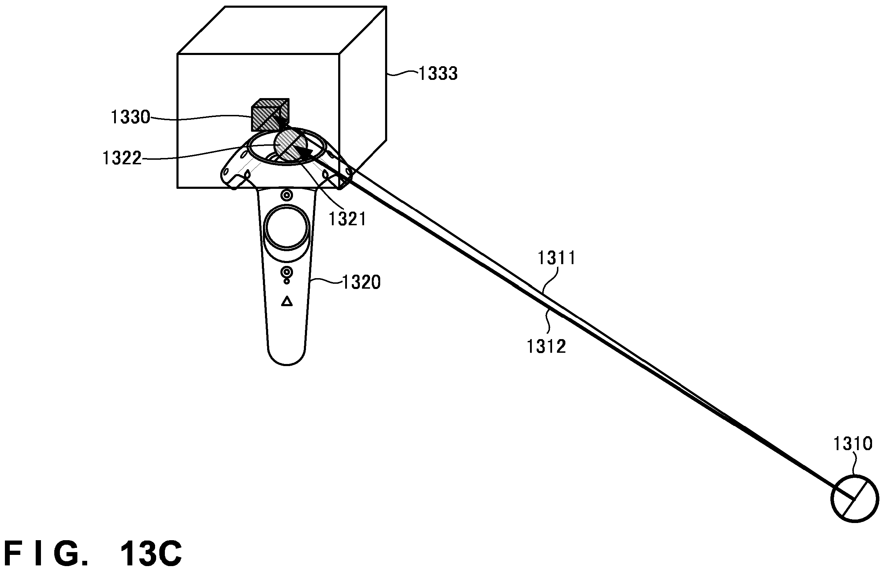

Fig. 13C is a view for explaining the outline of the operation of the rehabilitation assistance system according to this example embodiment. In this example embodiment, the gradation at the center of the object 1330 is darkened to form a reactive portion so no deviation occurs between the assumed target distance 1311 and the exercise distance 1312 of the avatar image 1320. Then, the gradation of the portion around the reactive portion of the object 1330 is lightened. That is, the size of the object 1330 shown in Figs. 13A and 13B is made small, and the object 1330 is surrounded by a visual recognition support image 1333 larger than the object 1330. That is, the object 1330 and the visual recognition support image 1333 are displayed in a superimposed manner.

Viewed from the user 220, when the size of the object 1330 is made small, the object 1330 is difficult to see (the visibility lowers). However, to compensate for the decrease in the visibility, the visual recognition support image 1333 is arranged around the object 1330 that has become small.

For example, the length of one side of the object 1330 is set to 5.0 cm, the length of one side of the visual recognition support image 1333 is set to 20.0 cm, and the diameter of a sensor portion 1322 of the avatar image 1320 is set to 2.0 cm. Then, the error (deviation) between the target distance 1311 and the exercise distance 1312 decreases to about 2.0 cm.

This can make it possible to decrease the deviation (error) between the target distance 1311 and the exercise distance 1312 while preventing the visibility of the object 1330 from lowering due to the gradation difference and the size difference between the object 1330 and the visual recognition support image 1333. Additionally, as a secondary effect, the degree of experience obtained by bringing the avatar image 1320 into contact with the object 1330 increases. That is, the sensation of touching the object 1330 is clear for the user 220, and the joy in achieving the target of the rehabilitation also increases.

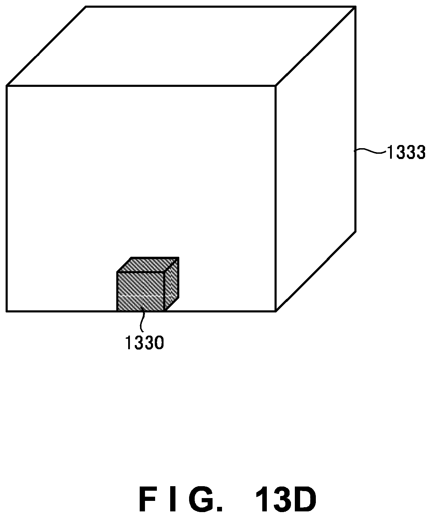

Fig. 13D is a view for explaining the arrangement position of a visual recognition support image in the rehabilitation assistance system according to this example embodiment. In Fig. 13C, the object 1330 serving as the target image is displayed so as to be included in the visual recognition support image 1333, and is also arranged near the center of the visual recognition support image 1333.

However, as shown in Fig. 13D, the object 1330 may be arranged near the lower side of the visual recognition support image 1333 on the near side. That is, the object 1330 may be arranged on the near side viewed from the user 220. In this way, the object 1330 can be arranged at any position in the visual recognition support image 1333 as long as it is displayed inside the visual recognition support image 1333. When the size of the object 1330 is made small, and the deviation between the target distance 1311 and the exercise distance 1312 is decreased, the visibility of the object 1330 lowers. Hence, to improve the visibility of the object 1330, the visual recognition support image 1333 larger than the object 1330 is displayed around the object 1330, thereby compensating for the decrease in the visibility of the object 1330. Note that the visual recognition support image 1333 used to improve the visibility of the object 1330 is not limited to a cube, as shown here, obtained by increasing the magnification of the cubic object 1330.

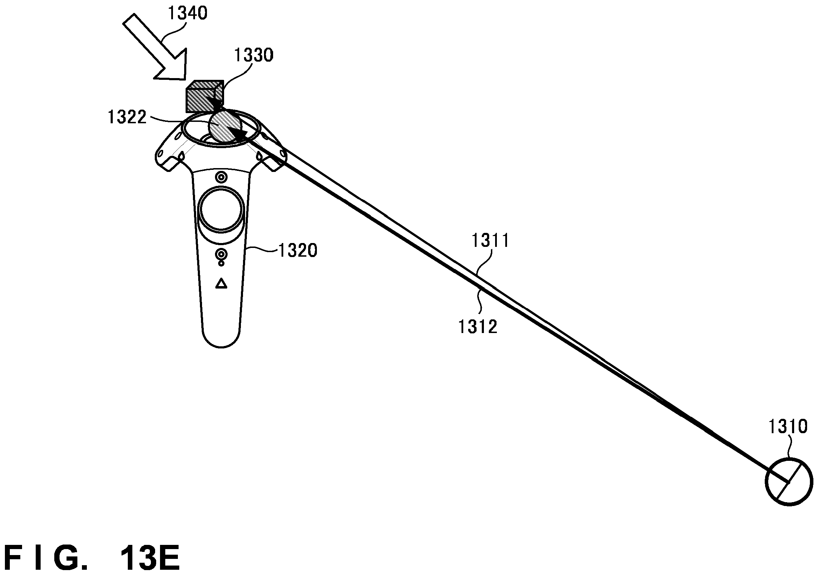

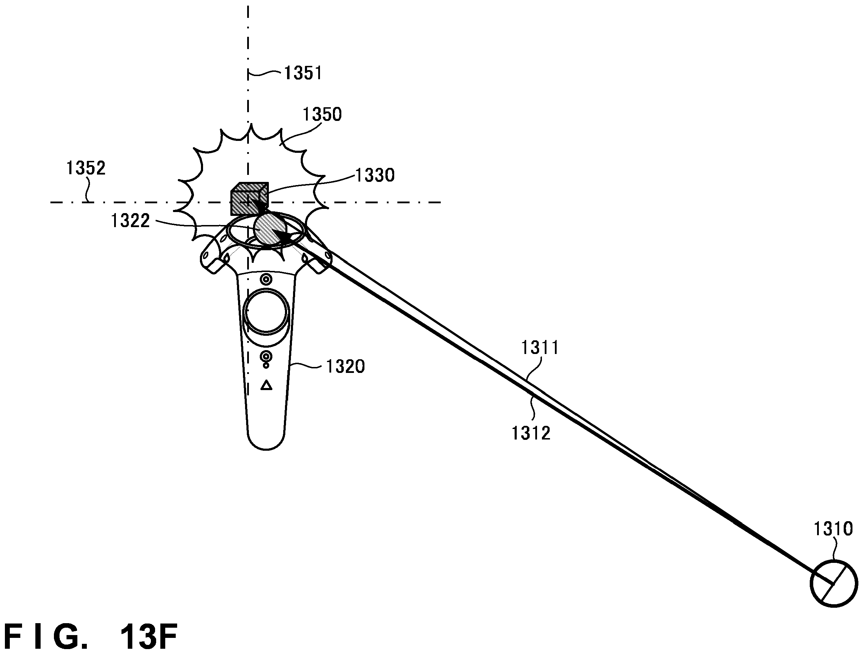

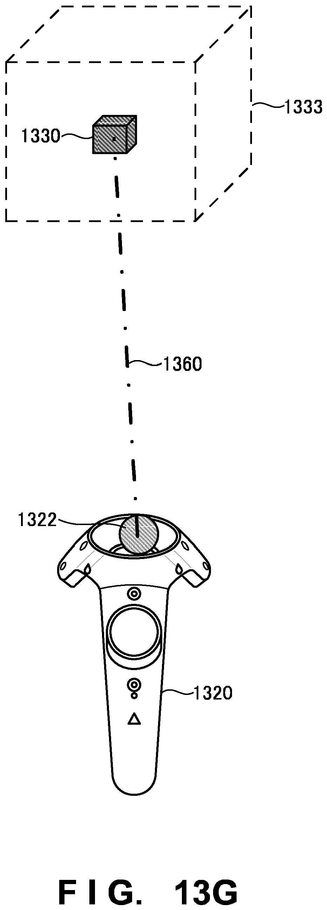

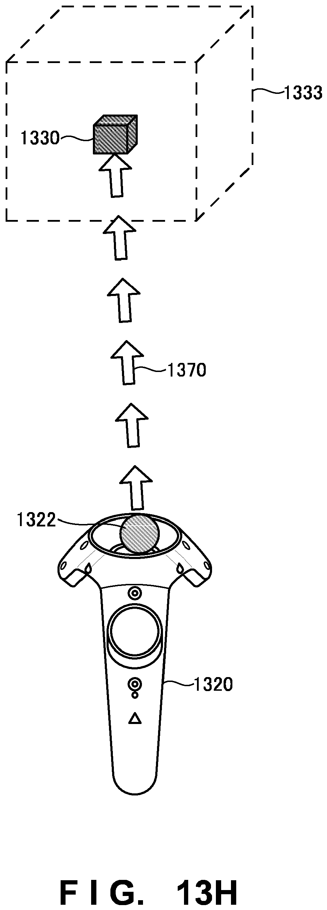

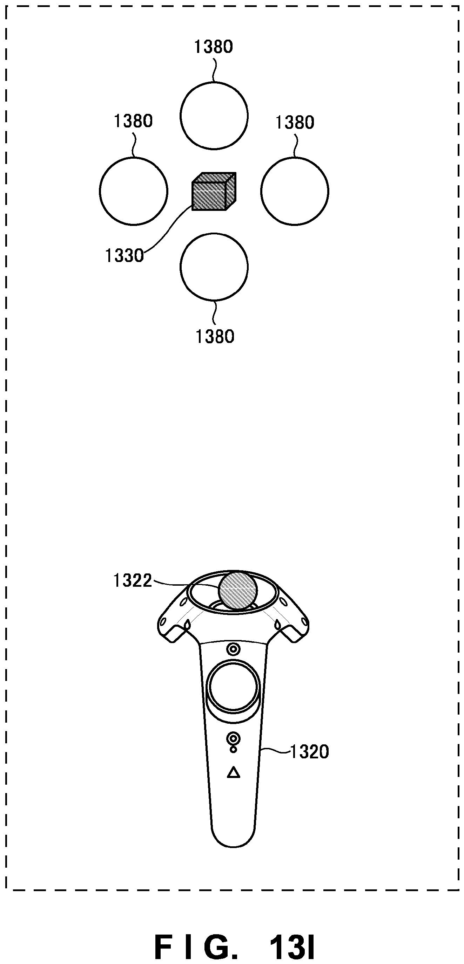

Other shapes of the visual recognition support image 1333 will be described next with reference to Figs. 13E to 13I. Fig. 13E is a view for explaining another example of the visual recognition support image in the rehabilitation assistance system according to this example embodiment. Fig. 13F is a view for explaining still another example of the visual recognition support image in the rehabilitation assistance system according to this example embodiment. Fig. 13G is a view for explaining still another example of the visual recognition support image in the rehabilitation assistance system according to this example embodiment. Fig. 13H is a view for explaining still another example of the visual recognition support image in the rehabilitation assistance system according to this example embodiment. Fig. 13I is a view for explaining still another example of the visual recognition support image in the rehabilitation assistance system according to this example embodiment.

As shown in Fig. 13E, a visual recognition support image 1340 may have, for example, an arrow shape representing the existence position of the object 1330. The object 1330 is not included in the arrow-shaped visual recognition support image 1340. That is, the object 1330 serving as the target image and the visual recognition support image 1340 are not displayed in a superimposed manner, and the visual recognition support image 1340 is displayed outside the object 1330. In this way, when the arrow-shaped visual recognition support image 1340 is used, the user 220 can easily recognize that the object 1330 exists at the tip of the arrow.

As shown in Fig. 13F, a visual recognition support image 1350 may have a shape for attracting the attention of the user 220. Note that the shape for attracting the attention of the user 220 is not limited to the shape shown in Fig. 13F and may be, for example, a star shape, a cross shape, a polygonal shape, or the like. In addition, a vertical line 1351 and a horizontal line 1352 may be displayed together to indicate that the object 1330 is arranged at the intersection of the vertical line 1351 and the horizontal line 1352.

As shown in Fig. 13G, a visual recognition support image 1360 may be an alternate long and short dashed line extending from the sensor portion 1322 of the avatar image 1320 to the object 1330. Note that the visual recognition support image 1360 is not limited to the alternate long and short dashed line and may be, for example, a straight line, an alternate long and two short dashed line, a dotted line, or the like.

Using the alternate long and short dashed line of the visual recognition support image 1360 as a guideline, the user 220 moves the line of sight along the alternate long and short dashed line and visually recognizes the object 1330, thereby recognizing the existence position of the object 1330. Furthermore, when the avatar image 1320 is moved along the alternate long and short dashed line, the user can make the avatar image 1320 touch the object 1330. Note that when the visual recognition support image 1333 is displayed together with a visual recognition support image 1360, the visibility of the object 1330 further improves.

As shown in Fig. 13H, the visual recognition support image 1370 may have a plurality of arrows arranged on a straight line from the sensor portion 1322 to the object 1330. Using the plurality of arrows as a guideline, the user 220 moves the line of sight along the plurality of arrows and visually recognizes the object 1330, thereby recognizing the existence position of the object 1330. Furthermore, when the avatar image 1320 is moved along the plurality of arrows, the user can make the avatar image 1320 touch the object 1330. Note that when the cubic visual recognition support image 1333 is displayed together with the visual recognition support image 1370, the visibility of the object 1330 further improves.

As shown in Fig. 13I, a plurality of spherical visual recognition support images 1380 are arranged at positions on the upper, lower, left, and right sides of the object 1330. That is, in Fig. 13I, the plurality of spherical visual recognition support images 1380 are arranged around the object 1330 such that the object 1330 is arranged at the center of the four visual recognition support images 1380. Note that the shape of the visual recognition support image 1380 is not limited to the spherical shape and may be, for example, a triangular shape, a rectangular shape, a polygonal shape, a star shape, or the like.

Fig. 13J is a view for explaining still another example of the visual recognition support image in the rehabilitation assistance system according to this example embodiment. The rehabilitation assistance server changes the size of the visual recognition support image 1333 displayed on a display unit 1402 in accordance with the degree of progress of rehabilitation of the user 220. For example, the rehabilitation assistance server displays the large visual recognition support image 1333 at the initial stage of the rehabilitation. In a state in which the rehabilitation of the user 220 has progressed, the size of the visual recognition support image 1333 is reduced in accordance with the degree of progress of rehabilitation.

In addition, in an embodiment not falling under the scope of the claims, the rehabilitation assistance server may change the size of the visual recognition support image 1333 not in accordance with the degree of progress of rehabilitation of the user 220 but in accordance with, for example, the eyesight of the user 220. That is, the rehabilitation assistance server displays the large visual recognition support image 1333 for the user 220 with poor eyesight, and displays the small visual recognition support image 1333 for the user 220 with relatively good eyesight. In this way, the rehabilitation assistance server may display the visual recognition support image having a size according to the eyesight of the user 220.

Additionally,, for example, if the user 220 has dementia, the rehabilitation assistance server may display the visual recognition support image 1333 having a size according to the degree of progress of dementia or the cognitive function. Note that the size of the visual recognition support image 1333 may be changed automatically by the rehabilitation assistance server, or may be changed manually by an operator such as a doctor who operates the rehabilitation assistance system and changed by the user 220.

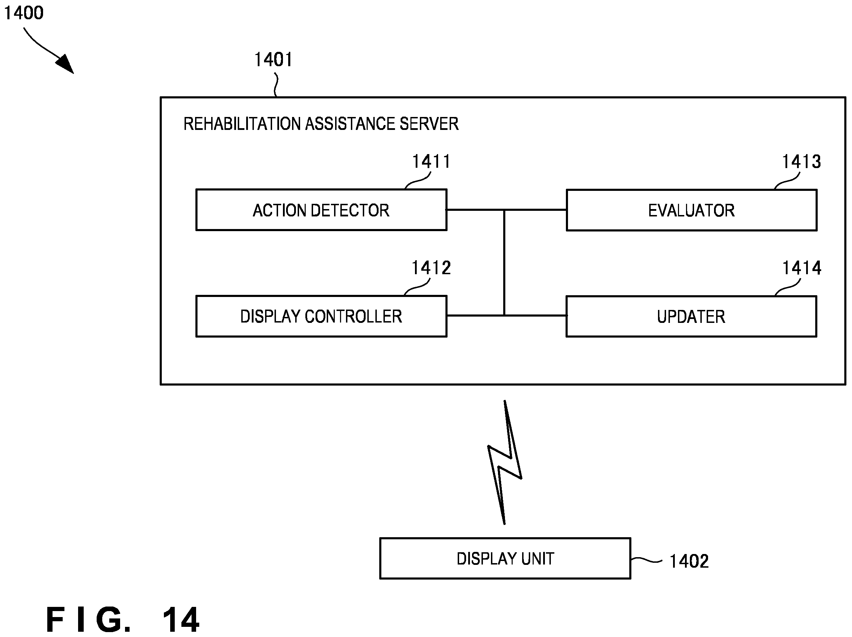

Fig. 14 is a block diagram for explaining the arrangement of the rehabilitation assistance system according to this example embodiment. A rehabilitation assistance system 1400 includes a rehabilitation assistance server 1401 and the display unit 1402. Note that the elements included in the rehabilitation assistance system 1400 are not limited to these. The rehabilitation assistance server 1401 includes an action detector 1411, a display controller 1412, an evaluator 1413, and an updater 1414.

The action detector 1411 acquires the position of a controller in the hand of the user 220 and the position of a head mounted display or the like worn by the user 220, and detects the motion (rehabilitation action) of the user 220 based on changes in the acquired positions.

The display controller 1412 causes the display unit 1402 to display the avatar image 1320 that moves in accordance with the detected rehabilitation action, the target image representing the target of the rehabilitation action, and at least one visual recognition support image 1333 used to improve the visibility of the target image.

The display controller 1412 displays the target image and the visual recognition support image 1333 in a superimposed manner. For example, the size of the target image is made smaller than the size of the visual recognition support image 1333, and the target image is displayed such that it is included in the visual recognition support image 1333.

The display controller 1412 may display the target image, for example, near the center of the visual recognition support image 1333. In addition, the display controller 1412 may display the target image not near the center of the visual recognition support image 1333 but at a position included in the visual recognition support image 1333 and on a side close to the avatar image 1320, that is, on the near side when viewed from the user 220.

The display controller 1412 may identifiably display the object 1330 and the visual recognition support image 1333. More specifically, for example, the gradation of the object 1330 is displayed darker than the gradation of the visual recognition support image 1333. Since the object 1330 is displayed darker, a contrast difference is generated with respect to the visual recognition support image 1333 displayed lighter, and the user 220 can reliably recognize the object 1330. Note that how to apply gradation to the object 1330 and the visual recognition support image 1333 is not limited to the method described here. For example, gradation may be applied such that even the user 220 with poor eyesight can reliably identify the object 1330 and the visual recognition support image 1333.

In addition, the display controller 1412 displays the object 1330 and the visual recognition support image 1333 in different colors so as to identifiably display the object 1330 and the visual recognition support image 1333. The display controller 1412 applies, for example, a dark color to the object 1330 and a light color to the visual recognition support image 1333. However, the combination (pattern) of applied colors is not limited to this. For example, a combination of colors that allow even the user 220 with color anomaly (color blindness) to reliably identify the object 1330 and the visual recognition support image 1333 may be used. Furthermore, the display controller 1412 may perform coloring capable of coping with the users 220 of various types such as weak eyesight, narrowing of visual field, and color anomaly. Note that the colors to be applied to the object 1330 and the visual recognition support image 1333 may be selected by the user 220 or may be selected by an operator such as a doctor.

Note that the gradations and colors of the object 1330 and the visual recognition support image 1333 have been described here. The gradations and colors may similarly be changed for the other visual recognition support images 1340, 1350, 1360, 1370, and 1380 as well.

Furthermore, the display controller 1412 controls the change of the display of the visual recognition support image 1333 in accordance with at least one of the eyesight of the user 220 and the evaluation result of the evaluator 1413. For example, the display controller 1412 changes the size of the visual recognition support image 1333 in accordance with the eyesight of the user 220, the degree of progress of the rehabilitation of the user 220, the degree of progress of the dementia of the user 220, or the like.

The evaluator 1413 compares the rehabilitation action detected by the action detector 1411 and the target position represented by the object 1330 serving as the target image displayed by the display controller 1412 and evaluates the rehabilitation ability of the user 220.

The updater 1414 updates the target position represented by the object 1330 in accordance with the evaluation result of the evaluator 1413.

The display unit 1402 displays the target image, the visual recognition support image, and the like under the control of the display controller 1412. The display unit 1402 is a head mounted display, a display, a screen, or the like but is not limited to these.

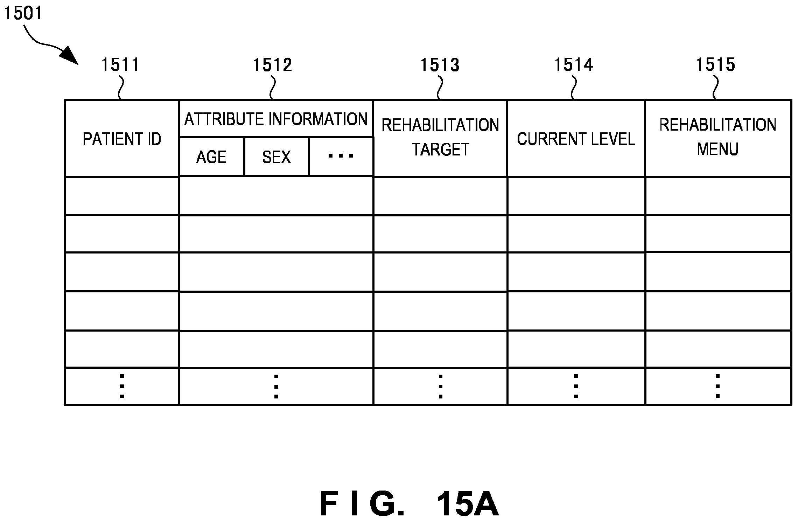

Fig. 15A is a view for explaining an example of a patient table provided in the rehabilitation assistance server included in the rehabilitation assistance system according to this example embodiment. A patient table 1501 stores attribute information 1512, a rehabilitation target 1513, a current level 1514, and a rehabilitation menu 1515 in association with a patient ID (Identifier) 1511. The patient ID 1511 is an identifier used to identify a patient. The attribute information 1512 is information representing attributes such as the age and sex of the patient. The rehabilitation target 1513 is data representing which part of the body of the patient is the target of rehabilitation, for example, data representing a body part such as an arm or a leg.

The current level 1514 is data representing the current rehabilitation level of the patient. That is, the current level 1514 is data representing the degree of progress or the like of the rehabilitation of the patient. The data is data dividing rehabilitation stages from the initial stage to the final stage into a plurality of ranks, for example, A rank, B rank, and the like. Note that the rehabilitation level division method is not limited to this. The rehabilitation menu 1515 is information concerning the menu of rehabilitation that the patient should undergo.

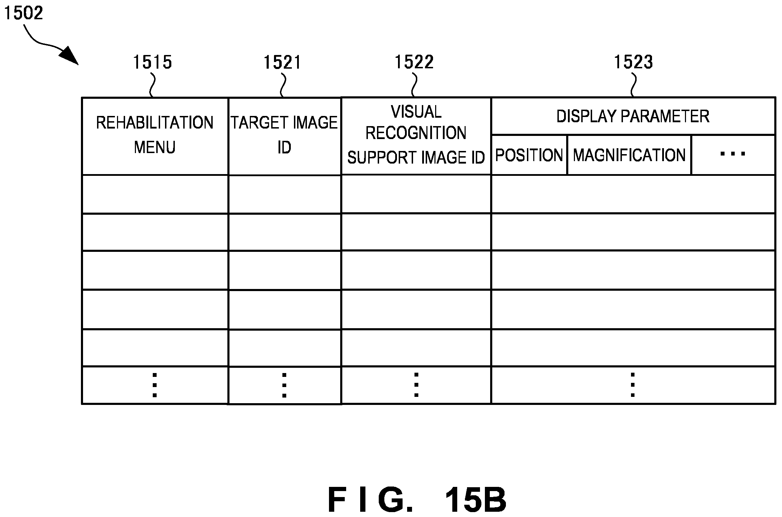

Next, Fig. 15B is a view for explaining an example of a display parameter table provided in the rehabilitation assistance server included in the rehabilitation assistance system according to this example embodiment. A display parameter table 1502 stores a target image ID 1521, a visual recognition support image ID 1522, and a display parameter 1523 in association with the rehabilitation menu 1515.

The target image ID 1521 is an identifier used to identify the object 1330 to be displayed on the display unit 1402. The visual recognition support image ID 1522 is an identifier used to identify the visual recognition support image 1333, 1340, 1350, 1360, 1370, or 1380 to be displayed on the display unit 1402. The display parameter 1523 is a parameter necessary for displaying the object 1330 or the visual recognition support image 1333, 1340, 1350, 1360, 1370, or 1380 on the display unit 1402. The display parameter 1523 includes, for example, pieces of information such as a position and a magnification. However, the pieces of information included in the display parameter 1523 are not limited to these.

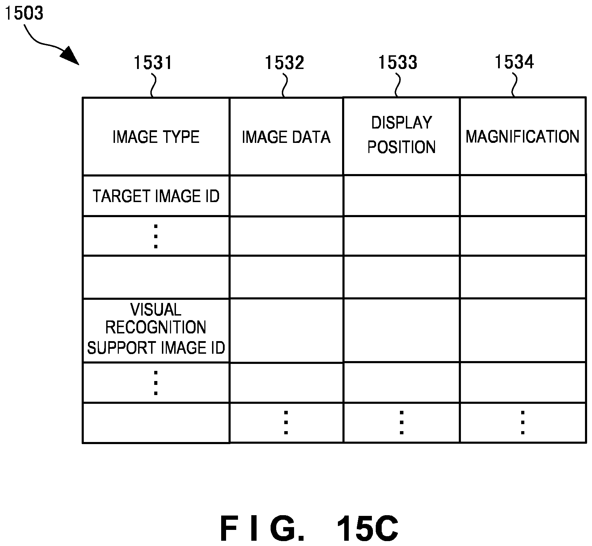

Fig. 15C is a view for explaining an example of an image table provided in the rehabilitation assistance server included in the rehabilitation assistance system according to this example embodiment. An image table 1503 stores image data 1532, a display position 1533, and a magnification 1534 in association with an image type 1531. Note that the items stored in the image table 1503 are not limited to these.

The image type 1531 is information for discriminating whether the image to be displayed is a target image or a visual recognition support image. The image data 1532 is the image data of the object 1330 or the visual recognition support image 1333 to be displayed on the display unit 1402 and includes image data of various image file formats. The display position 1533 is data representing a position in the display unit 1402 at which an image should be displayed, and is, for example, the data of a set of (X-coordinate position, Y-coordinate position, Z-coordinate position). The magnification 1534 is data used to decide the size to display the object 1330, the visual recognition support image 1333, or the like on the display unit 1402.

The rehabilitation assistance server 1401 refers to the tables 1501, 1502, and 1503 and displays the visual recognition support images 1333, 1340, 1350, 1360, 1370, and 1380 on the display unit 1402.

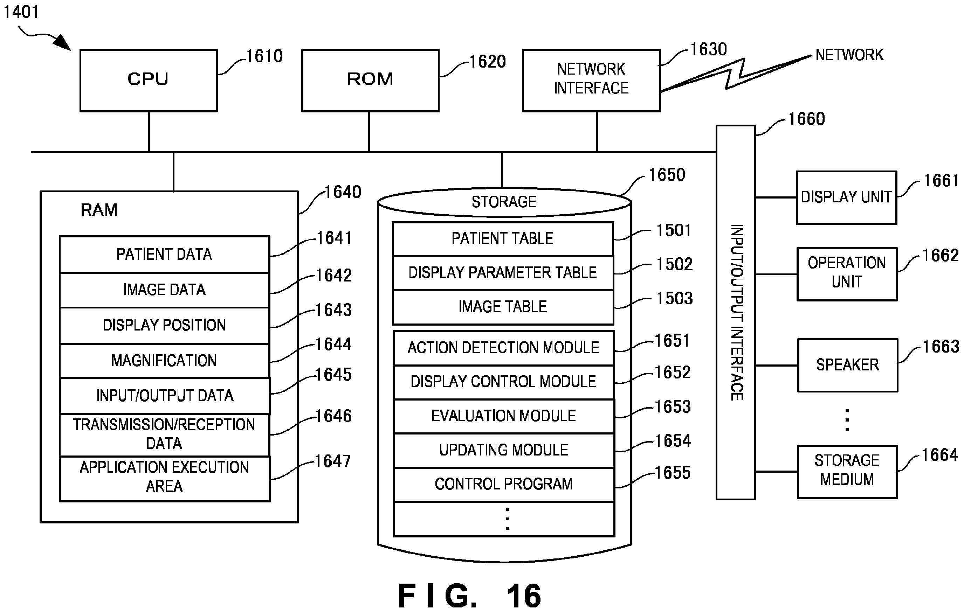

Fig. 16 is a block diagram for explaining the hardware arrangement of the rehabilitation assistance server included in the rehabilitation assistance system according to this example embodiment. A CPU (Central Processing Unit) 1610 is a processor for arithmetic control and executes a program, thereby implementing the functional components of the rehabilitation assistance server 1401 shown in Fig. 14. A ROM (Read Only Memory) 1620 stores permanent data such as initial data and a program, and other programs. A network interface 1630 communicates with another device or the like via a network. Note that the CPU 1610 is not limited to one CPU and may include a plurality of CPUs or a GPU (Graphics Processing Unit) for image processing. In addition, the network interface 1630 preferably includes a CPU independent of the CPU 1610 and writes or reads transmission/reception data in or from an area of a RAM (Random Access Memory) 1640. In addition, it is preferable to provide a DMAC (Direct Memory Access Controller) (not shown) configured to transfer data between the RAM 1640 and a storage 1650. In addition, an input/output interface 1660 preferably includes a CPU independent of the CPU 1610 and writes or reads input/output data in or from an area of the RAM 1640. Hence, the CPU 1610 recognizes that data is received from or transferred to the RAM 1640 and processes the data. In addition, the CPU 1610 prepares a processing result in the RAM 1640 and leaves subsequent transmission or transfer to the network interface 1630, the DMAC, or the input/output interface 1660.

The RAM 1640 is a random access memory used by the CPU 1610 as a work area for temporary storage. In the RAM 1640, an area to store data necessary for implementation of this example embodiment is allocated. Patient data 1641 is data concerning a patient who undergoes rehabilitation using the rehabilitation assistance system. Image data 1642 is the data of the object 1330 serving as a target image or the visual recognition support image 1333 to be displayed on the display unit 1402. A display position 1643 is data representing a position in the display unit 1402 at which the object 1330 or the visual recognition support image 1333 should be displayed. A magnification 1644 is data representing the size to display an image such as the object 1330 or the visual recognition support image 1333 on the display unit 1402. These data are read out from, for example, the patient table 1501, the display parameter table 1502, and the image table 1503.

Input/output data 1645 is data input/output via the input/output interface 1660. Transmission/reception data 1646 is data transmitted/received via the network interface 1630. In addition, the RAM 1640 includes an application execution area 1647 used to execute various kinds of application modules.

The storage 1650 stores databases, various kinds of parameters, and following data and programs necessary for implementation of this example embodiment. The storage 1650 stores the patient table 1501, the display parameter table 1502, and the image table 1503. The patient table 1501 is a table that manages the relationship between the patient ID 1511 and the attribute information 1512 and the like shown in Fig. 15A. The display parameter table 1502 is a table that manages the relationship between the rehabilitation menu 1515 and the display parameter 1523 and the like shown in Fig. 15B. The image table 1503 is a table that manages the relationship between the image type 1531 and the image data 1532 and the like shown in Fig. 15C.

The storage 1650 further stores an action detection module 1651, a display control module 1652, an evaluation module 1653, and an updating module 1654.

The action detection module 1651 is a module configured to detect the rehabilitation action of the user 220. The display control module 1652 is a module configured to display the avatar image 1320, the object 1330 serving as a target image, the visual recognition support image 1333 used to improve the visibility of the object 1330, and the like on the display unit 1402. The evaluation module 1653 is a module configured to evaluate the rehabilitation ability of the user 220. The updating module 1654 is a module configured to update the target position represented by the target image in accordance with the evaluation result. The modules 1651 to 1654 are loaded into the application execution area 1647 of the RAM 1640 and executed by the CPU 1610. A control program 1655 is a program configured to control the entire rehabilitation assistance server 1401.

The input/output interface 1660 interfaces input/output data to/from an input/output device. A display unit 1661 and an operation unit 1662 are connected to the input/output interface 1660. In addition, a storage medium 1664 may further be connected to the input/output interface 1660. Furthermore, a speaker 1663 that is a voice output unit, a microphone that is a voice input unit, or a GPS (Global Positioning System) position determiner may be connected. Note that programs and data concerning general-purpose functions or other implementable functions of the rehabilitation assistance server 1401 are not illustrated in the RAM 1640 and the storage 1650 shown in Fig. 16.

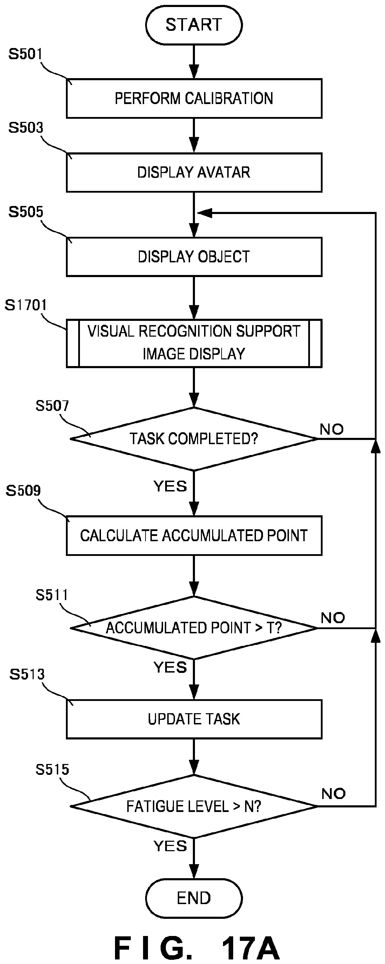

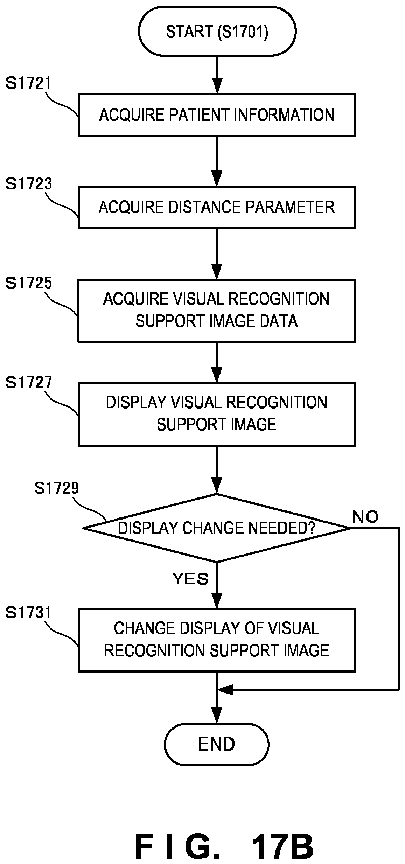

Fig. 17A is a flowchart for explaining the processing procedure of the rehabilitation assistance server included in the rehabilitation assistance system according to this third example embodiment. Fig. 17B is a flowchart for explaining the processing procedure of visual recognition support image display of the rehabilitation assistance server included in the rehabilitation assistance system according to this example embodiment. These flowcharts are executed by the CPU 1610 using the RAM 1640 and implement the functional components of the rehabilitation assistance server 1401 shown in Fig. 14.

In step S1701, the rehabilitation assistance server 1401 causes the display unit 1402 or the like to display a visual recognition support image.

In step S1721, the rehabilitation assistance server 1401 acquires patient information representing the attribute of the patient who undergoes rehabilitation using the rehabilitation assistance system 1400 and what kind of rehabilitation menu the patient should undergo.

In step S1723, the rehabilitation assistance server 1401 acquires display parameters necessary for displaying, on the display unit 1402, the visual recognition support image 1333 and the like to be displayed on the display unit 1402. The display parameters to be acquired are parameters concerning the position and magnification of the visual recognition support image 1333 and the like.

In step S1725, the rehabilitation assistance server 1401 acquires image data of the visual recognition support image 1333. In step S1727, the rehabilitation assistance server 1401 displays the visual recognition support image 1333 and the like on the display unit 1402.

In step S1729, the rehabilitation assistance server 1401 judges whether the display of the visual recognition support image 1333 and the like needs to be changed. If the display change is not needed (NO in step S1729), the rehabilitation assistance server 1401 ends the processing. If the display change is needed (YES in step S1729), the rehabilitation assistance server 1401 advances to the next step.

In step S1731, the rehabilitation assistance server 1401 changes the size of the visual recognition support image 1333 in accordance with the eyesight of the user 220 or the evaluation result of the rehabilitation ability of the user 220.

According to this example embodiment, even if the size of the target image is made small to reduce the deviation between the target distance and the exercise distance, the effect of rehabilitation can be increased by making the target distance and the exercise distance close while maintaining the visibility of the target image. In addition, since the sensation of touching the target image is clear for the user, the user can experience feeling of satisfaction in achieving the target.

### [Fourth Example Embodiment]

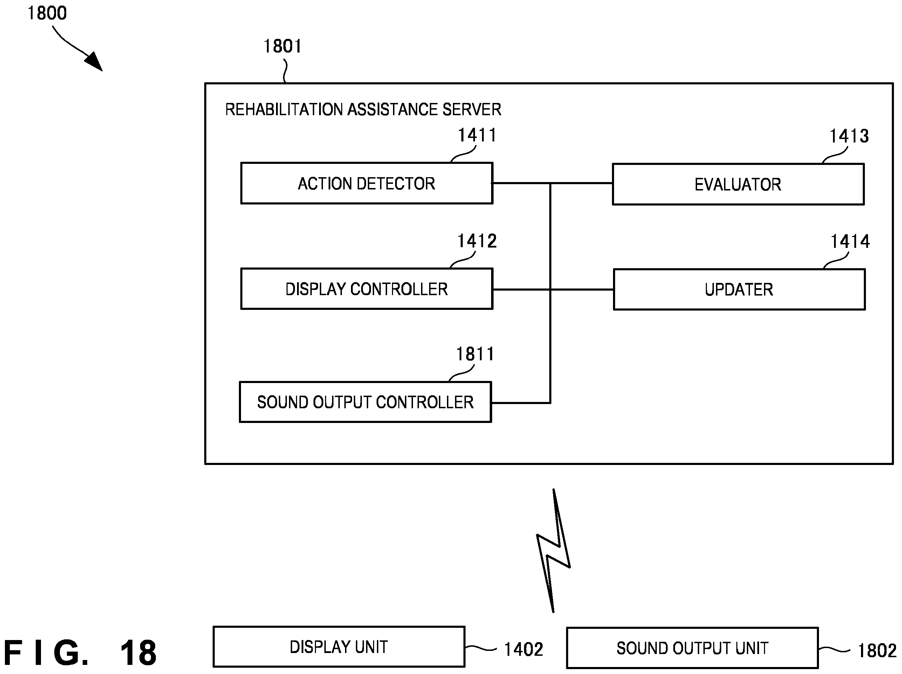

A rehabilitation assistance system according to the fourth example embodiment of the present invention will be described next with reference to Figs. 18 to 21. Fig. 18 is a block diagram for explaining the arrangement of the rehabilitation assistance system according to this example embodiment. The rehabilitation assistance system according to this example embodiment is different from the above-described third example embodiment in that the rehabilitation assistance system includes a sound output unit. The rest of the components and operations is the same as in the second example embodiment and the third example embodiment. Hence, the same reference numerals denote the same components and operations, and a detailed description thereof will be omitted.

A rehabilitation assistance system 1800 includes a rehabilitation assistance server 1801 and a sound output unit 1802. The rehabilitation assistance server 1801 includes a sound output controller 1811. The sound output controller 1811 controls output of a sound in accordance with the positional relationship between an object 1330 serving as a target image and an avatar image 1320. The sound whose output is controlled by the sound output controller 1811 is output from the sound output unit 1802.

For example, when the object 1330 falls downward from above, the sound output controller 1811 outputs a sound based on the distance, that is, the positional relationship between the object 1330 and the avatar image 1320.

The output sound may be changed to a sound of a higher frequency as the distance between the object 1330 and the avatar image 1320 decreases, that is, the object 1330 moves close to the avatar image 1320. In addition, similarly, the output sound may be changed to a sound of a lower frequency as the distance between the object 1330 and the avatar image 1320 increases, that is, the object 1330 moves away from the avatar image 1320. That is, an acoustic effect like the Doppler effect for observing a difference in the frequency of the sound (wave) in accordance with the distance between the object 1330 (sound source) and the avatar image 1320 (user 220 (observer)) may be expressed. Note that instead of changing the frequency of the output sound, the volume of the output sound may be increased/decreased in accordance with the distance between the object 1330 and the avatar image 1320.

In addition, the position of the object 1330 may be instructed to the user 220 by outputting a sound from the sound output controller 1811. That is, the position of the object 1330 is instructed using the sense of hearing of the user 220.

For example, consider a case in which the user 220 wears a headphone when using the rehabilitation assistance system 1800. When the object 1330 serving as a target image is located on the right side of the avatar image 1320 (user 220), the rehabilitation assistance server 1801 outputs a sound from the right ear side of the headphone. Similarly, when the object 1330 is located on the left side of the avatar image 1320 (user 220), the rehabilitation assistance server 1801 outputs a sound from the left ear side of the headphone. This allows the user 220 to judge, based on the direction of the sound, whether the object 1330 is located on the right side or left side of the user 220. In addition, when the object 1330 is located in front of the avatar image 1320 (user 220), the rehabilitation assistance server 1801 outputs a sound from both sides of the headphone.

In the above description, the position of the object 1330 is instructed using the sense of sight or the sense of hearing of the user 220. However, one of the five senses other than the sense of sight and the sense of hearing, for example, the sense of taste, the sense of touch, or the sense of smell may be used to instruct the position of the object 1330 to the user 220.

For example, a sensor is placed on the tongue of the user 220 to cause the user 220 to feel a taste according to the position of the object 1330. Alternatively, the controller in the hand of the user 220 or the headphone or head mounted display worn by the user 220 may be vibrated. That is, the position of the object 1330 may be instructed using the sense of touch of the user 220.

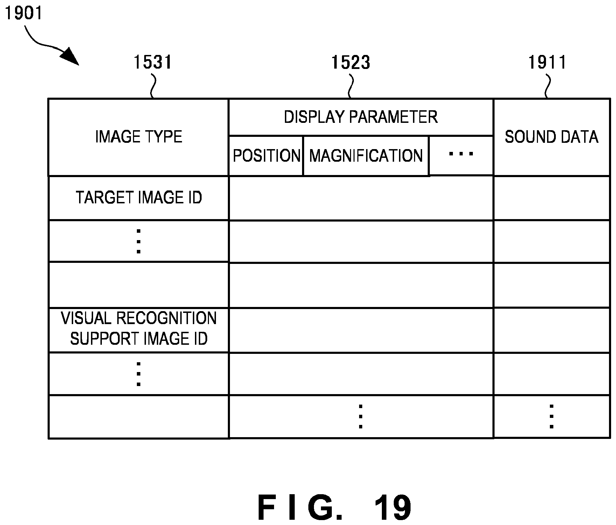

Fig. 19 is a view for explaining an example of a sound table provided in the rehabilitation assistance server included in the rehabilitation assistance system according to this example embodiment. A sound table 1901 stores sound data 1911 in association with an image type 1531. The rehabilitation assistance server 1801 controls the sound to be output by referring to the sound table 1901.

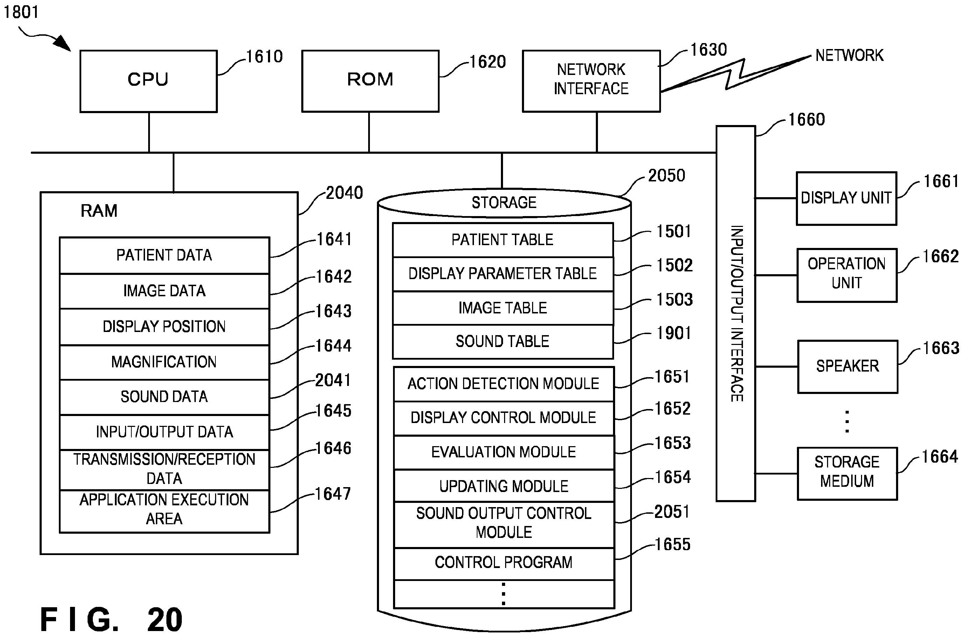

Fig. 20 is a view for explaining the hardware arrangement of the rehabilitation assistance server included in the rehabilitation assistance system according to this example embodiment. A RAM 2040 is a random access memory used by a CPU 1610 as a work area for temporary storage. In the RAM 2040, an area to store data necessary for implementation of this example embodiment is allocated. Sound data 2041 is data concerning a sound to be output. This data is read out from, for example, the sound table 1901.

A storage 2050 stores databases, various kinds of parameters, and following data and programs necessary for implementation of this example embodiment. The storage 2050 stores the sound table 1901. The sound table 1901 is the table that manages the relationship between the image type 1531 and the sound data 1911 shown in Fig. 19.

The storage 2050 further stores a sound output control module 2051. The sound output control module 2051 is a module configured to control output of a sound in accordance with the positional relationship between the object 1330 serving as a target image and the avatar image 1320. The module 2051 is loaded into an application execution area 1647 of the RAM 2040 and executed by the CPU 1610. Note that programs and data concerning general-purpose functions or other implementable functions of the rehabilitation assistance server 1801 are not illustrated in the RAM 2040 and the storage 2050 shown in Fig. 20.

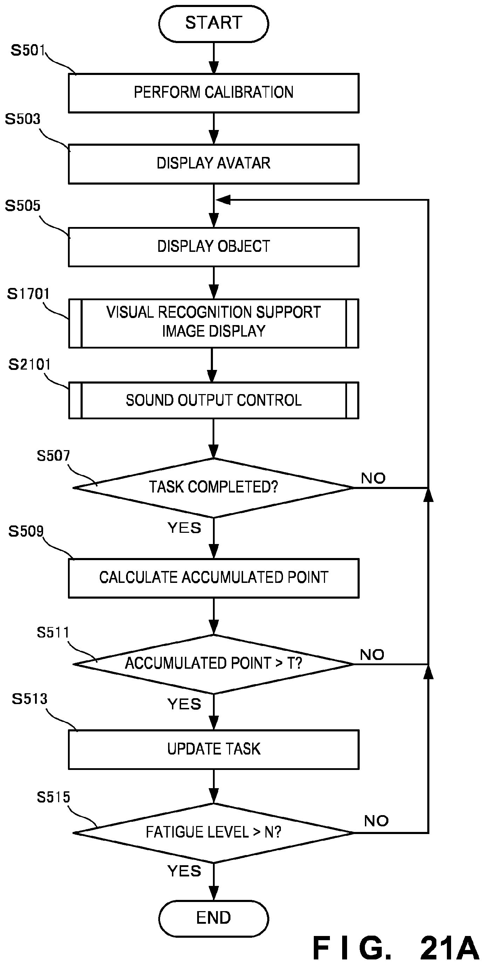

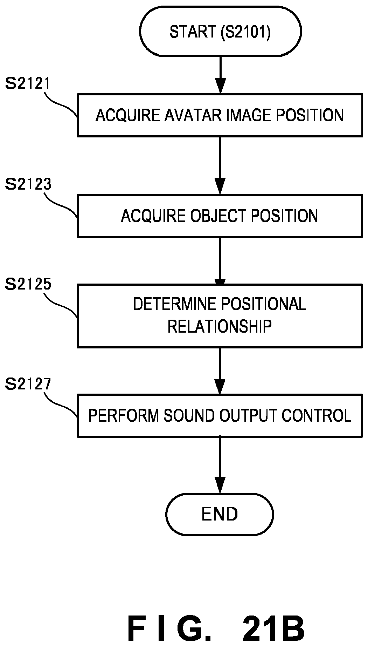

Fig. 21A is a flowchart for explaining the processing procedure of the rehabilitation assistance server included in the rehabilitation assistance system according to this example embodiment. Fig. 21B is a flowchart for explaining the processing procedure of sound output control of the rehabilitation assistance server included in the rehabilitation assistance system according to this example embodiment. These flowcharts are executed by the CPU 1610 using the RAM 2040 and implement the functional components of the rehabilitation assistance server 1801 shown in Fig. 18.

In step S2101, the rehabilitation assistance server 1801 controls output of a sound. In step S2121, the rehabilitation assistance server 1801 acquires the position of the avatar image 1320. In step S2123, the rehabilitation assistance server 1801 acquires the position of the object 1330. In step S2125, the rehabilitation assistance server 1801 determines the positional relationship between the avatar image 1320 and the object 1330. In step S2127, the rehabilitation assistance server 1801 controls the output of a sound in accordance with the determined positional relationship.

According to this example embodiment, since the rehabilitation is executed using the sense of hearing in addition to the sense of sight of the user, the user can more easily visually recognize the object, and the effect obtained by the rehabilitation can further be enhanced. In addition, the user can grasp the position of the object not only by the sense of sight but also by the sense of hearing. Furthermore, since a sound is output, even a user with poor eyesight can undergo the rehabilitation according to this example embodiment.

### [Fifth Example Embodiment]

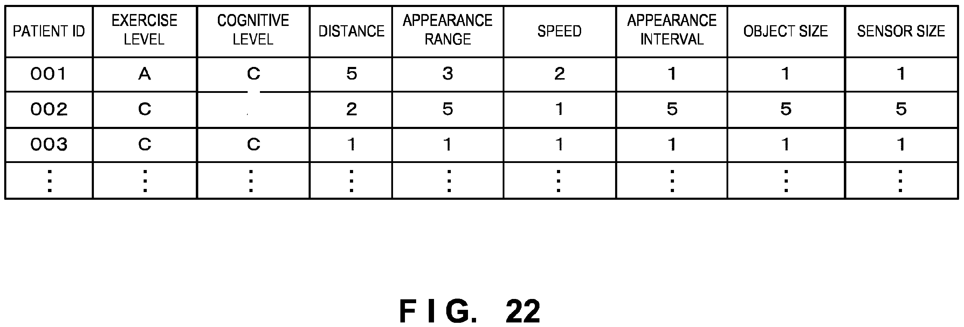

A system according to the fifth example embodiment of the present invention will be described next with reference to Figs. 22 to 24. A rehabilitation assistance system according to this example embodiment is different from the above-described third example embodiment in that a target is made definite by a plurality of parameters. The rest of the components and operations is the same as in the third example embodiment. Hence, the same reference numerals denote the same components and operations, and a detailed description thereof will be omitted.

Fig. 22 is a view showing the contents of a target DB 216 according to this example embodiment in detail. As shown in Fig. 22, a target to be currently achieved in rehabilitation is set for each patient. First, the exercise level and cognitive level of a patient are individually determined as the attributes of the patient. If the exercise level or cognitive level is high, the level is evaluated as A. A low level is evaluated as C, and a medium level is evaluated as B. For example, in a case of patient ID 001, the exercise level is high, but the cognitive level is low. In this case, the distance up to the object, that is, the distance to stretch out the hand at maximum is long (here, for example, level 5 in five levels), the object appearance range is narrow to some extent (here, for example, level 3), and the speed of the motion of the object is low (here, for example, level 2). In addition, the object appearance interval is long (here, for example, level 1), and both the object size and the sensor size are large (here, for example, level 1).

On the other hand, in a case of patient ID 002, the exercise level is low, but the cognitive level is high. In this case, the distance to the object, that is, the distance to stretch out the hand at maximum is short (here, for example, level 2 in five levels), the object appearance range is wide (here, for example, level 5), and the speed of the motion of the object is low (here, for example, level 1). On the other hand, the object appearance interval is short (here, for example, 5 in five levels), and both the object size and the sensor size are small (here, for example, 5 in five levels).

In a case of patient ID 003, both the exercise level and the cognitive level are low. In this case, the distance to the object, that is, the distance to stretch out the hand at maximum is short (here, for example, level 1 in five levels), the object appearance range is narrow (here, for example, level 1), and the speed of the motion of the object is low (here, for example, level 1). In addition, the object appearance interval is long (here, for example, 1 in five levels), and both the object size and the sensor size are large (here, for example, 1 in five levels).

In this way, the parameters are variously changed in accordance with the attributes of the patient.

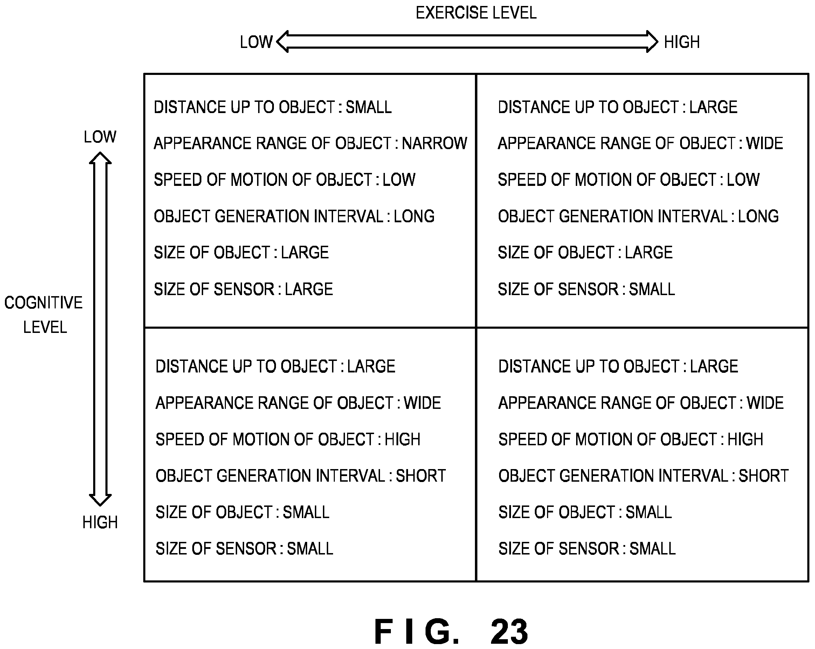

In general, the relationship between the motor function, the cognitive function, and various kinds of parameters is expected as shown in Fig. 23. However, the rehabilitation assistance system according to the present invention does not set parameters limited to this relationship, and can search for a rehabilitation intensity suitable for each patient by changing various kinds of parameters (distance, range, speed, interval, and size) in accordance with the state and ability of the patient.

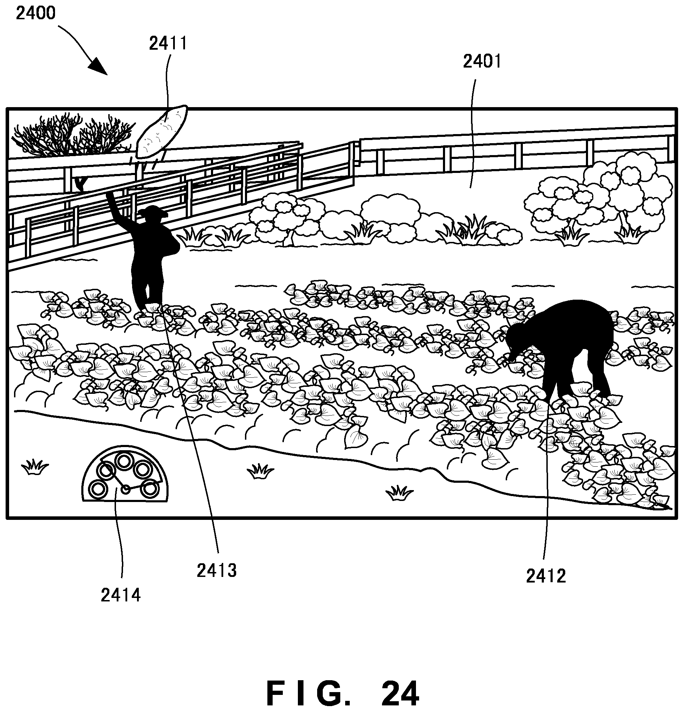

Fig. 24 shows a screen example 2400 that a display controller 212 displays on a head mounted display 233 in this example embodiment. The display controller 212 displays an object 2411 superimposed on a background image 2401. The display controller 212 displays the object 2411 having the shape of a sweet potato while gradually changing its display position and size such that the object 2411 seems to fall downward from above the user 220. Here, an image 2412 of a state in which a farmer is bending forward is displayed as a preliminary state to the appearance of the object 2411. Upon recognizing the farmer 2412 bending forward, the user predicts that the object 2411 then appears from the direction of the farmer. In addition, since a farmer 2413 throws the object 2411 upward, the user spontaneously performs an operation of following the object 2411 with eyes and looking up. That is, it is possible to make not a linguistic instruction but an instruction that makes the user to be spontaneously conscious of the upper side.

After that, the user 220 moves controllers 234 and 235 in accordance with the position of the falling object 2411 to move an avatar image 311 (not shown in Fig. 24) having the shape of a basket. When the falling object 2411 enters the basket, the mission is completed, and the requested rehabilitation action is completed. In a case in which the object 2411 cannot be put in the basket, a child helping collection of the sweet potato may be displayed to relieve the mental shock or stress of the user. Note that an auxiliary indicator 2414 may be displayed to show the appearance position of the farmer to the user.

In this example as well, it is possible to set a rehabilitation intensity appropriate for the user by changing various kinds of parameters (the distance to the falling sweet potato, the range of appearance of the farmer, the falling speed of the sweet potato, the interval to throw the sweet potato by the farmer, and the size of the basket) in accordance with the motor function and the cognitive function of the user.

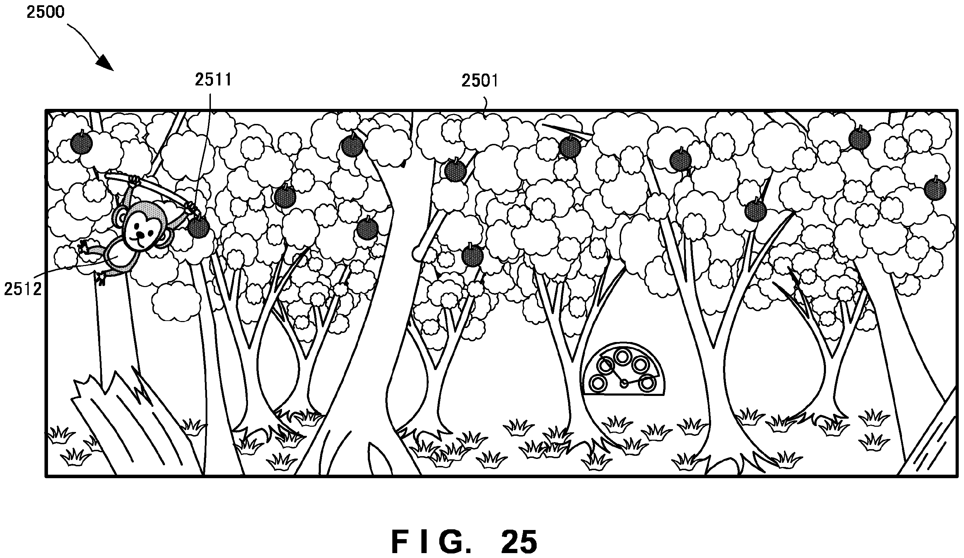

Fig. 25 shows another screen example 2500 that the display controller 212 displays on the head mounted display 233 in this example embodiment. Here, the display controller 212 displays an object 2511 superimposed on a background image 2501. In this example, the display controller 212 displays the object 2511 having the shape of an apple while gradually changing its display position and size such that the object 2511 seems to fall downward from above the user 220. An image 2512 of a monkey shaking a tree is displayed as a preliminary state to the fall of the object 2511 having the shape of an apple. Upon recognizing the monkey, the user predicts that the object 2511 then falls from the direction of the monkey. After that, the user 220 moves the controllers 234 and 235 in accordance with the position of the falling object 2511 to move the avatar image 311 (not shown in Fig. 25) having the shape of a basket. When the falling object 2511 enters the basket, the mission is completed, and the requested rehabilitation action is completed. In a case in which the object 2511 cannot be put in the basket as well, a child helping collection of the apple may be displayed to relieve the mental shock or stress of the user.

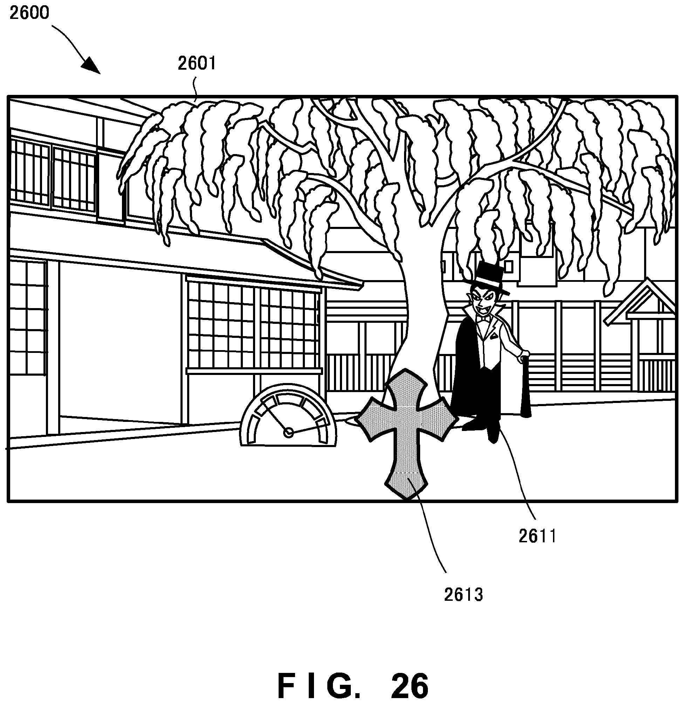

Fig. 26 shows still another screen example 2600 that the display controller 212 displays on the head mounted display 233 in this example embodiment. Here, the display controller 212 displays an object 2611 superimposed on a background image 2601. In this example, the display controller 212 displays the object 2611 having the shape of Dracula while gradually changing its display position and size such that the object 2611 approaches from the far side to the user 220. The user 220 moves the controllers 234 and 235 in accordance with the position of approaching Dracula to move an image 2613 having the shape of a cross. When the cross hits Dracula, the mission is completed, and the requested rehabilitation action is completed. In a case in which the cross cannot hit Dracula as well, a helping child may be displayed to relieve the mental shock or stress of the user.

According to the above-described examples, it is possible to give a task to both the motor function and the cognitive function of the user. For example, a task to the cognitive function of the user can be given by displaying a preliminary state such as a farmer bending forward or a monkey appearing, and a task to the motor function of the user can be given by changing the distance, direction, speed, and the like of an object. That is, the patient is caused to perform both a motor rehabilitation action of stretching out an arm and a cognitive rehabilitation action of predicting the next appearance position of an object and moving the line of sight. This makes it possible to perform more effective rehabilitation.

Note that the visual recognition support image described in the third example embodiment may be additionally displayed for the object in each of Figs. 24 to 26. In this case, the size of the outline of the visual recognition support image may be changed in accordance with the cognitive function of the patient. In addition, stepwise evaluation (good when touching only the outline and very good when touching the center) may be done in a case in which the avatar image serving as a sensor touches only the visual recognition support image (outline) and in a case in which the avatar image touches the object.

### [Other Example Embodiments]

While the invention has been described with reference to example embodiments thereof, the invention is not limited to these example embodiments. For example, the display device is not limited to the head mounted display but may be a large screen. The controller is not limited to a grip type but may be a wearable sensor.

While the invention has been particularly shown and described with reference to example embodiments thereof, the invention is not limited to these example embodiments. It will be understood by those of ordinary skill in the art that various changes in form and details may be made therein without departing from the spirit and scope of the present invention as defined by the claims.

The present invention is applicable to a system including a plurality of devices or a single apparatus. The present invention is also applicable even when an information processing program for implementing the functions of example embodiments is supplied to the system or apparatus directly or from a remote site. Hence, the present invention also incorporates the program installed in a computer to implement the functions of the present invention by the computer, a medium storing the program, and a WWW (World Wide Web) server that causes a user to download the program. Especially, the present invention incorporates at least a non-transitory computer readable medium storing a program that causes a computer to execute processing steps included in the above-described example embodiments.

## Claims

1. A rehabilitation assistance system comprising:
an action detector (1411) configured to detect a first rehabilitation action of a user:
a display controller (1412) configured to control a display unit to display an avatar image (1320) that moves in a three-dimensional virtual space in accordance with the detected first rehabilitation action, further configured to control the display unit to display a target object (1330) representing a target of the first rehabilitation action in the three-dimensional virtual space and further configured to control the display unit to display a visual recognition support image (1333) that improves the recognizability of the target object by being displayed such that the target object is surrounded by the visual recognition support image being larger than the target object;
an evaluator (1413) configured to evaluate a rehabilitation ability of the user by comparing the position of the avatar with the position of the target object, wherein the first rehabilitation action is judged as completed when the avatar touches the target object, and
an updater (1414) configured to update the position of the target object in accordance with an evaluation result by said evaluator,
wherein the size of the visual recognition support image changes in accordance with the evaluation result,
wherein said action detector is further configured to detect a second rehabilitation action during the first rehabilitation action, and wherein
said evaluator is further configured to evaluate the rehabilitation ability based on both the first rehabilitation action and the second rehabilitation action.

2. The rehabilitation assistance system according to claim 1, wherein stepwise evaluation is done in a case in which the avatar image touches only the visual recognition support image and in a case in which the avatar image touches the target object.

3. The rehabilitation assistance system according to any one of claims 1 to 2, wherein said updater changes a characteristic and an intensity of the rehabilitation by changing, as a setting parameter, at least one of a distance up to the target object, a direction of the target object, a speed of a motion of the target object, an interval of appearance of the target object, a size of the target object, and a size of the avatar image.

4. The rehabilitation assistance system according to claim 1 or 2, wherein said display controller is configured to display a question image superimposed on the avatar image and the target object, and
said evaluator is configured to evaluate the rehabilitation ability of the user using an answer to the question image as the second rehabilitation action.

5. The rehabilitation assistance system according to any one of claims 1 to 4, wherein said display controller is configured to display an outline of the target object and an outline of a visual recognition support image that improves visibility of the target object.

## Patentansprüche

1. Rehabilitationsassistenzsystem, umfassend:
einen Aktionsdetektor (1411), der so konfiguriert ist, dass er eine erste Rehabilitationsaktion eines Benutzers erkennt;
eine Anzeigesteuerung (1412), die so konfiguriert ist, dass sie eine Anzeigeeinheit steuert, um ein Avatarbild (1320) anzuzeigen, das sich in einem dreidimensionalen virtuellen Raum in Übereinstimmung mit der erkannten ersten Rehabilitationsaktion bewegt, ferner so konfiguriert ist,
dass sie die Anzeigeeinheit steuert, um ein Zielobjekt (1330) anzuzeigen, das ein Ziel der ersten Rehabilitationsaktion in dem dreidimensionalen virtuellen Raum darstellt, und ferner konfiguriert ist,
um die Anzeigeeinheit zu steuern, um ein visuelles Erkennungsunterstützungsbild (1333) anzuzeigen, das die Erkennbarkeit des Zielobjekts verbessert, indem es so angezeigt wird, dass das Zielobjekt von dem visuellen Erkennungsunterstützungsbild umgeben ist, das größer als das Zielobjekt ist,
einen Evaluator (1413), der so konfiguriert ist, dass er eine Rehabilitationsfähigkeit des Benutzers durch Vergleichen der Position des Avatars mit der Position des Zielobjekts evaluiert, wobei die erste Rehabilitationsaktion als abgeschlossen beurteilt wird, wenn der Avatar das Zielobjekt berührt; und
einen Updater (1414), der so konfiguriert ist, dass er die Position des Zielobjekts gemäß einem Evaluierungsergebnis des Evaluators aktualisiert,
wobei sich die Größe des visuellen Erkennungsunterstützungsbildes gemäß dem Auswertungsergebnis ändert,
wobei der Aktionsdetektor ferner so konfiguriert ist, dass er eine zweite Rehabilitationsaktion während der ersten Rehabilitationsaktion erkennt, und wobei
der Evaluator ferner so konfiguriert ist, dass er die Rehabilitationsfähigkeit sowohl auf der Grundlage der ersten Rehabilitationsaktion als auch der zweiten Rehabilitationsaktion evaluiert.

2. Rehabilitationsassistenzsystem nach Anspruch 1, wobei eine schrittweise Evaluierung in einem Fall durchgeführt wird, in dem das Avatar-Bild nur das visuelle Erkennungsunterstützungsbild berührt, und in einem Fall, in dem das Avatar-Bild das Zielobjekt berührt.

3. Rehabilitationsassistenzsystem nach einem der Ansprüche 1 bis 2, wobei derUpdater eine Eigenschaft und eine Intensität der Rehabilitation ändert, indem er als Einstellungsparameter mindestens einen der folgenden Parameter ändert: eine Entfernung bis zum Zielobjekt, eine Richtung des Zielobjekts, eine Geschwindigkeit einer Bewegung des Zielobjekts, ein Intervall des Erscheinens des Zielobjekts, eine Größe des Zielobjekts und eine Größe des Avatarbildes.

4. Rehabilitationsassistenzsystem nach Anspruch 1 oder 2, wobei die Anzeigesteuerung so konfiguriert ist, dass sie ein Fragebild anzeigt, das das Avatarbild und das Zielobjekt überlagert, und die Auswerteeinrichtung so konfiguriert ist, dass sie die Rehabilitationsfähigkeit des Benutzers unter Verwendung einer Antwort auf das Fragebild als zweite Rehabilitationsmaßnahme auswertet.

5. Rehabilitationsassistenzsystem nach einem der Ansprüche 1 bis 4, wobei die Anzeigesteuerung so konfiguriert ist, dass sie einen Umriss des Zielobjekts und einen Umriss eines visuellen Erkennungsunterstützungsbildes anzeigt, das die Sichtbarkeit des Zielobjekts verbessert.

## Revendications

1. Un système d'aide à la rééducation comprenant :
un détecteur d'action (1411) configuré pour détecter une première action de rééducation d'un utilisateur ;
un dispositif (1412) de commande d'affichage configuré pour commander une unité d'affichage afin d'afficher une image d'avatar (1320) qui se déplace dans un espace virtuel tridimensionnel conformément à la première action de rééducation détectée, configuré en outre pour commander à l'unité d'affichage d'afficher un objet cible (1330) représentant une cible de la première action de rééducation dans l'espace virtuel tridimensionnel et configuré en outre pour commander à l'unité d'affichage d'afficher une image de support de reconnaissance visuelle (1333) qui améliore la reconnaissabilité de l'objet cible en étant affichée de telle sorte que l'objet cible est entouré par l'image de support de reconnaissance visuelle qui est plus grande que l'objet cible ;
un évaluateur (1413) configuré pour évaluer une capacité de rééducation de l'utilisateur en comparant la position de l'avatar avec la position de l'objet cible, la première action de rééducation étant jugée comme étant terminée lorsque l'avatar touche l'objet cible, et
un moyen de mise à jour (1414), configuré pour mettre à jour la position de l'objet cible conformément à un résultat d'évaluation par ledit évaluateur,
la taille de l'image de support de reconnaissance visuelle changeant en fonction du résultat de l'évaluation,
ledit détecteur d'action étant en outre configuré pour détecter une deuxième action de rééducation pendant la première action de rééducation, et
ledit évaluateur est en outre configuré pour évaluer la capacité de rééducation sur la base à la fois de la première action de rééducation et de la deuxième action de rééducation.

2. Le système d'aide à la rééducation selon la revendication 1, dans lequel l'évaluation étape par étape est effectuée dans le cas où l'image de l'avatar touche uniquement l'image support de reconnaissance visuelle et dans le cas où l'image de l'avatar touche l'objet cible.

3. Le système d'aide à la rééducation selon l'une quelconque des revendications 1 à 2, dans lequel ledit moyen de mise à jour modifie une caractéristique et une intensité de la rééducation en modifiant, en tant que paramètre de réglage, au moins un parmi une distance jusqu'à l'objet cible, une direction de l'objet cible, une vitesse de mouvement de l'objet cible, un intervalle d'apparition de l'objet cible, une taille de l'objet cible et une taille de l'image de l'avatar.

4. Le système d'aide à la rééducation selon la revendication 1 ou la revendication 2, dans lequel ledit dispositif de commande d'affichage est configuré pour afficher une image de question superposée à l'image de l'avatar et à l'objet cible, et
ledit évaluateur est configuré pour évaluer la capacité de rééducation de l'utilisateur en utilisant une réponse à l'image de question comme deuxième action de rééducation.

5. Le système d'aide à la rééducation selon l'une quelconque des revendications 1 à 4, dans lequel ledit contrôleur d'affichage est configuré pour afficher un contour de l'objet cible et un contour d'une image support de reconnaissance visuelle qui améliore la visibilité de l'objet cible.
